(19) Europäisches Patentamt
European Patent Office
Office européen des brevets

(11) **EP 3 684 245 B1**

(12) **EUROPEAN PATENT SPECIFICATION**

(45) Date of publication and mention of the grant of the patent:
**10.11.2021 Bulletin 2021/45**

(51) Int Cl.:
***A61B 5/00*** *(2006.01)* ***A61B 5/06*** *(2006.01)*
***H04B 13/00*** *(2006.01)*

(21) Application number: **18769714.9**

(22) Date of filing: **17.09.2018**

(86) International application number:
**PCT/EP2018/075005**

(87) International publication number:
**WO 2019/057651 (28.03.2019 Gazette 2019/13)**

(54) **DETERMINING AN ORIENTATION OF A WEARABLE DEVICE**

BESTIMMUNG EINER AUSRICHTUNG EINER AM KÖRPER TRAGBAREN VORRICHTUNG

DÉTERMINATION D'UNE ORIENTATION D'UN DISPOSITIF VESTIMENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.09.2017 EP 17192268**

(43) Date of publication of application:
**29.07.2020 Bulletin 2020/31**

(73) Proprietor: **Koninklijke Philips N.V.**
**5656 AG Eindhoven (NL)**

(72) Inventors:
• **VISWESWARA, Ashoka, Sathanur**
**5656 AE Eindhoven (NL)**
• **MEFTAH, Mohammed**
**5656 AE Eindhoven (NL)**
• **JOHNSON, Mark, Thomas**
**5656 AE Eindhoven (NL)**

(74) Representative: **Philips Intellectual Property & Standards**
**High Tech Campus 5**
**5656 AE Eindhoven (NL)**

(56) References cited:
**WO-A1-2016/181321 US-A1- 2013 338 448**
**US-A1- 2014 155 721**

**Description**

TECHNICAL FIELD OF THE INVENTION

The invention relates to the field of devices and, in particular, to determining an orientation of a wearable device when placed on a body.

BACKGROUND TO THE INVENTION

Wearable devices (such as wearable patches) play a pivotal role in medical care and rehabilitation procedures. For example, wearable devices can comprise sensors for monitoring a subject wearing the device and/or medication dispensers for dispensing medication to the subject wearing the device. Often wearable devices comprising sensors form part of a body area network through which medical professionals can acquire data on the subject from a remote location. The data can, for example, include the vital signs of the subject. The wearable devices comprising sensors are usually placed at a location on the body of the subject that is appropriate for the relevant information to be acquired. Similarly, wearable devices comprising medication dispensers are usually placed at a location on the body of the subject that is appropriate for the medication to be administered. For these reasons, the placement of wearable devices is typically performed by a medical professional (such as a nurse or doctor) in a medical environment (such as a hospital).

However, wearable devices are now used in a wider variety of situations. For example, wearable devices comprising sensors can be used for monitoring subjects in low acuity settings (such as in a general ward or at home) and can even be used by subjects to monitor themselves. It is beneficial to improve monitoring in low acuity settings to enable earlier discharge from high acuity settings and to also reduce the mortality rate by detecting deterioration as early as possible. A problem that exists with the use of wearable devices in low acuity settings is that most wearable devices need to be replaced every few days (for example, due to battery depletion, hygiene, degradation of adhesives, or skin irritation) and the subjects themselves or informal caregivers often then need to replace the wearable device.

A difficulty with this is that the placement of a wearable device at a correct orientation on the body of a subject is often key for the performance and/or the proper operation of the wearable devices. For example, a wearable device in the form of an electrocardiography (ECG) patch needs to be placed at an accurate orientation on the chest of the subject. However, the placement of wearable devices at a correct orientation can be challenging, especially for an untrained user and particularly where the user is elderly as the user may have problems with eyesight, dexterity, bending, or other issues.

WO 2016/181321 A1 discloses a wireless physiological device for acquiring and processing physiological signals.

US 2013/0338448 A1 discloses an adherent device which comprises an adhesive patch with at least two electrodes and an accelerometer. The accelerometer can be used to determine an orientation of the at least two measurement electrodes on a patient. By determining the orientation of the electrodes of the patch on the patient, physiologic measurements with the at least two electrodes can be adjusted and/or corrected in response to the orientation of the patch on the patient.

There exist methods that provide feedback for the proper placement of a wearable device, which can help when the wearable device needs to be replaced. For example, US 2013/0116533 A1 discloses a system for providing quantitative feedback (such as the morphological similarity between the acquired ECG waveform and the pre-stored ECG templates, as well as the signal-to-noise ratio) to the subject to guide the proper placement of a disposable unit on the body. Other existing methods that aid in the proper placement of a wearable device involve a simple ink marker on the wearable device that indicates the correct orientation of the wearable device relative the body. However, these existing methods require the (possibly untrained) user to identify the orientation of the wearable device on the body based on their own observations and analysis of complex signal morphologies acquired from the wearable device or from reference markers on the wearable device. This can be complicated and prone to errors. There is thus still a need to determine the orientation of a wearable device in a manner that is simplified and yet reliable.

SUMMARY OF THE INVENTION

It would thus be valuable to have an improved method and device for determining an orientation of a wearable device, which overcome the existing problems.

Therefore, according to a first aspect, there is provided a wearable device operable to determine an orientation of the wearable device, when placed on a body. The wearable device comprises a plurality of electrode pairs located at different positions. At least two of the plurality of electrode pairs are configured to receive a signal transmitted from a reference device using the body as a transmission medium. The wearable device also comprises a processor configured to determine a property associated with the signal received at the at least two electrode pairs and compare the determined property to a corresponding reference property. The determined property is indicative of an orientation of the wearable device with respect to the reference device and the corresponding reference property is indicative of a predetermined orientation for the wearable device on the body. The processor is also configured to determine, from the comparison, whether the wearable device is in the predetermined orientation on the body.

In some embodiments, the determined property associated with the signal received at the at least two electrode pairs ma be indicative of a distance of one of the at least two electrode pairs from the reference device relative to a distance of at least one other of the at least two electrode pairs from the reference device.

In some embodiments, the property associated with the signal received at the at least two electrode pairs may comprise any one or more of: a phase angle difference between the signal received at the at least two electrode pairs, a time of flight of the signal received at one of the at least two electrode pairs relative to a time of flight of the signal received at at least one other of the at least two electrode pairs, and an amplitude of the signal received at one of the at least two electrode pairs relative to an amplitude of the signal received at at least one other of the at least two electrode pairs.

In some embodiments, the wearable device may be time synchronised with the reference device prior to the transmission of the signal from the reference device.

In some embodiments, the processor may be further configured to control a feedback component to render an output indicative of whether the wearable device is in the predetermined orientation on the body.

In some embodiments, the wearable device may further comprise at least one physiological characteristic sensor configured to obtain at least one physiological characteristic signal from the body.

According to a second aspect, there is provided a method of operating a wearable device to determine an orientation of the wearable device, when placed on a body. The wearable device comprises a plurality of electrode pairs located at different positions. The method comprises receiving, at at least two of the plurality of electrode pairs, a signal transmitted from a reference device using the body as a transmission medium, determining, by a processor of the wearable device, a property associated with the signal received at the at least two electrode pairs and comparing, by the processor, the determined property to a corresponding reference property. The determined property is indicative of an orientation of the wearable device with respect to the reference device and the corresponding reference property is indicative of a predetermined orientation for the wearable device on the body. The method also comprises determining, by the processor, from the comparison, whether the wearable device is in the predetermined orientation on the body.

According to a third aspect, there is provided a reference device to determine an orientation of a wearable device, when the wearable device is placed on a body. The wearable device comprises a plurality of electrode pairs located at different positions. The reference device comprises a receiver configured to receive signals transmitted from at least two of the plurality of electrode pairs of the wearable device through the body as a transmission medium. The reference device also comprises a processor configured to determine a property associated with the signals received at the receiver from the at least two electrode pairs and compare the determined property to a corresponding reference property. The determined property is indicative of an orientation of the wearable device with respect to the reference device and the corresponding reference property is indicative of a predetermined orientation for the wearable device on the body. The processor is also configured to determine, from the comparison, whether the wearable device is in the predetermined orientation on the body.

In some embodiments, the determined property associated with the signal received at the receiver may be indicative of a distance of one of the at least two electrode pairs from the reference device relative to a distance of at least one other of the at least two electrode pairs from the reference device.

In some embodiments, the property associated with the signals received at the receiver from the at least two electrode pairs may comprise any one or more of: a phase angle difference between the signals received at the receiver from the at least two electrode pairs, a time of flight of the signal received at the receiver from one of the at least two electrode pairs relative to a time of flight of the signal received at the receiver from at least one other of the at least two electrode pairs, and an amplitude of the signal received at the receiver from one of the at least two electrode pairs relative to an amplitude of the signal received at the receiver from at least one other of the at least two electrode pairs.

In some embodiments, the reference device may be time synchronised with the wearable device prior to the transmission of the signals from the at least two electrode pairs.

In some embodiments, the processor may be further configured to control a feedback component to render an output indicative of whether the wearable device is in a predefined orientation on the body.

In some embodiments, the reference device may be a device contactable with at least part of the body.

According to a fourth aspect, there is provided a method of operating a reference device to determine an orientation of a wearable device, when the wearable device is placed on a body. The wearable device comprises a plurality of electrode pairs located at different positions. The method comprises receiving, at a receiver of the reference device, signals transmitted from at least two of the plurality of electrode pairs of the wearable device through the body as a transmission medium, determining, by a processor of the reference device, a property associated with the signals received at the receiver from the at least two electrode pairs and comparing, by the processor, the determined property to a corresponding reference property. The determined property is indicative of an orientation of the wearable device with respect to the reference device and the corresponding reference property is indicative of a predetermined orientation for the wearable device on the body. The method also comprises determining, by the processor, from the comparison, whether the wearable device is in the predetermined orientation on the body.

According to a fifth aspect, there is provided a system comprising the wearable device as described above and the reference device configured to transmit the signal, or the reference device as described above and the wearable device comprising the at least two electrodes configured to transmit the signals.

According to a sixth aspect of the invention, there is provided a computer program product comprising a computer readable medium, the computer readable medium having computer readable code embodied therein, the computer readable code being configured such that, on execution by a suitable computer or processor, the computer or processor is caused to perform the method or the methods described above.

According to the aspects and embodiments described above, the limitations of existing techniques are addressed. In particular, according to the above-described aspects and embodiments, a determination of the orientation of the wearable device is made in a manner that is simplified and yet reliable. The above-described aspects and embodiments can be useful in facilitating or assisting in the placement of a wearable device in a predetermined orientation on the body of a subject.

There is thus provided an improved method and device for determining an orientation of a wearable device, which overcomes the existing problems. The invention is defined by appended claims 1-15.

BRIEF DESCRIPTION OF THE DRAWINGS

For a better understanding of the embodiments, and to show more clearly how they may be carried into effect, reference will now be made, by way of example only, to the accompanying drawings, in which:

Fig. 1 is a block diagram of a wearable device operable to determine an orientation of the wearable device according to an embodiment;
Fig. 2 is a block diagram of a wearable device operable to determine an orientation of the wearable device according to another embodiment;
Fig. 3 is an illustration of a system comprising a wearable device operable to determine an orientation of the wearable device according to an embodiment;
Fig. 4 is an illustration of a system comprising a wearable device operable to determine an orientation of the wearable device according to another embodiment;
Fig. 5 is a flow chart illustrating a method of operating a wearable device to determine an orientation of the wearable device according to an embodiment;
Fig. 6 is an illustration of a phase angle difference between signals received at a plurality of electrodes of a wearable device according to an embodiment;
Fig. 7 is an illustration of device signals according to an embodiment;
Fig. 8 is a block diagram of a reference device operable to determine an orientation of a wearable device according to an embodiment;
Fig. 9 is an illustration of a system comprising a reference device operable to determine an orientation of a wearable device according to an embodiment;
Fig. 10 is an illustration of a system comprising a reference device operable to determine an orientation of a wearable device according to another embodiment;
Fig. 11 is a flow chart illustrating a method of operating a reference device to determine an orientation of the wearable device according to an embodiment;
Fig. 12 is a flow chart illustrating a method of operating a wearable device or a reference device to determine the orientation of the wearable device according to an example embodiment; and
Fig. 13 is a flow chart illustrating a method of operating a wearable device or a reference device to determine the orientation of the wearable device according to another example embodiment.

DETAILED DESCRIPTION OF THE EMBODIMENTS

As noted above, there is provided an improved method and device for determining an orientation of a wearable device, which overcomes existing problems. In some of the embodiments that will be described herein, the wearable device itself can be operable to determine its orientation, when placed on a body. In other embodiments that will be described herein, a reference device can be operable to determine the orientation of the wearable device, when the wearable device is placed on a body. The body referred to herein can be a body of a subject, where the subject may be, for example, a patient, a user, or any other subject.

The reference device referred to herein can be any device that is contactable with at least part of the body. In some embodiments, for example, the reference device may be a wearable device or an on-body device. For example, the reference device can be a wrist-worn device (such as a watch or smartwatch), a neck-worn device (such as a pendant) a finger or thumb worn device (such as a ring), or any other type of wearable device or on-body device that is contactable with at least part of the body. In other embodiments, the reference device may be an off-body device, such as a device on which the body can be placed or a device that the body can touch. For example, the reference device can be a device on which the body can stand such as a weighing device (or a weighing scales), a chair, a bed, a tablet, a smart phone, a smart mirror, or any other device that is contactable with at least part of the body.

The wearable device referred to herein can be any device that is adapted to be placed on (or worn on) a body. For example, the wearable device can be an on-body device. In some embodiments, the wearable device may be in the form of a patch. In some embodiments, the wearable device may comprise an adhesive surface for adhering to the skin of the body. In embodiments where the reference device is also a wearable device, the reference device may take the same form as the wearable device such as that described here or may take a different form.

Although some examples of the form of the wearable device and the reference device have been provided, it will be understood that any other form of the wearable device and the reference device is also possible.

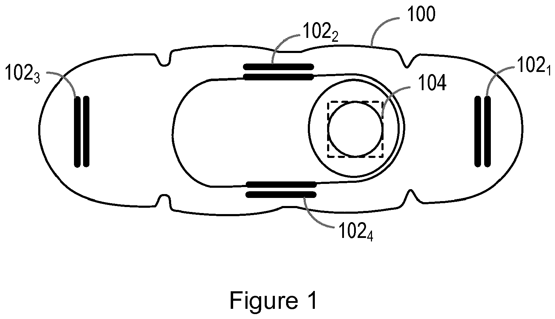

Fig. 1 illustrates an example of a wearable device 100 operable to determine an orientation of the wearable device 100, when placed on a body, according to an embodiment.

As illustrated in Fig. 1, the wearable device 100 comprises a plurality of electrode pairs $102i$, $102_2$, $102_3$, $102_4$ located at different positions. For example, in some embodiments, the plurality of electrode pairs $102i$, $102_2$, $102_3$, $102_4$ may be located at different positions around a perimeter of the wearable device 100. As illustrated in Fig. 1, for example, the plurality of electrode pairs $102i$, $102_2$, $102_3$, $102_4$ may be positioned on the periphery of the four sides of the wearable device 100 according to some embodiments. In some embodiments, one or more of the plurality of electrode pairs $102i$, $102_2$, $102_3$, $102_4$ may be located on (for example, attached or connected to) the wearable device 100. Alternatively or in addition, in some embodiments, one or more of the plurality of electrode pairs $102i$, $102_2$, $102_3$, $102_4$ may be located in (for example, integrated in or embedded in) the wearable device 100. In the example embodiment illustrated in Fig. 1, the wearable device 100 is illustrated as comprising four electrode pairs $102_1$, $102_2$, $102_3$, $102_4$. However, it will be understood that any other number of multiple electrode pairs is possible, such as two electrode pairs, three electrode pairs, five electrode pairs, six electrode pairs, or more electrode pairs.

In any of the embodiments of the wearable device 100 described herein, at least two of the plurality of electrode pairs $102i$, $102_2$, $102_3$, $102_4$ are configured to receive a signal transmitted from a reference device. In some embodiments, at least two of the plurality of electrode pairs $102i$, $102_2$, $102_3$, $102_4$ may also be configured to transmit a signal to a reference device and/or any other device. Essentially, the at least two electrode pairs $102_1$, $102_2$, $102_3$, $102_4$ can operate as a receiver (or a receiving antenna) and, optionally, also a transmitter (or a transmitting antenna) of the wearable device 100.

The at least two electrode pairs $102i$, $102_2$, $102_3$, $102_4$ are configured to receive a signal transmitted from a reference device using the body as a transmission medium (or, more specifically, a signal transmission medium). Thus, the signal transmitted from the reference device can be referred to as a body channel sensing (BCS) signal. The signal transmitted can be an electrical signal, such as an alternating current (AC) signal or an alternating voltage signal. The signal may be a signal of a certain frequency and amplitude. For example, the signal may be a radio frequency (RF) signal, such as a signal with a frequency in the range of 10 MHz to 150 MHz. This range of frequencies results in the body behaving as a waveguide for signal transmission and enables the determination of the orientation of the wearable device 100 with sufficient accuracy. In some embodiments, the at least two electrode pairs $102i$, $102_2$, $102_3$, $102_4$ may be in galvanic contact with the body (or, more specifically, the skin of the body). However, it will be understood that capacitive coupling between the at least two electrode pairs $102i$, $102_2$, $102_3$, $102_4$ and the body (or, more specifically, the skin of the body) is sufficient.

As illustrated in Fig. 1, the wearable device 100 also comprises a processor 104. The processor 104 controls the operation of the wearable device 100 and can implement the method in relation to the wearable device 100, which is described herein. The processor 104 can comprise one or more processors (such as one or more microprocessors MPUs), one or more processing units, one or more multi-core processors and/or one or more controllers (such as one or more microcontrollers MCU), that are configured or programmed to control the wearable device 100 in the manner described herein. In particular implementations, the processor 104 can comprise a plurality of software and/or hardware modules, each configured to perform, or that are for performing, individual or multiple steps of the method described herein in relation to the wearable device 100.

Briefly, the processor 104 of the wearable device 100 is configured to determine a property associated with the signal received at the at least two electrode pairs $102i$, $102_2$, $102_3$, $102_4$ and compare the determined property to a corresponding reference property. The processor 104 of the wearable device 100 is also configured to determine, from the comparison, whether the wearable device 100 is in the predetermined orientation on the body.

The determined property referred to herein is indicative of an orientation of the wearable device 100 with respect to the reference device. For example, the determined property referred to herein can correspond to a unique orientation of the wearable device 100 on the body. The corresponding reference property referred to herein is indicative of a predetermined orientation for the wearable device 100 on the body. For example, the corresponding reference property referred to herein can correspond to an orientation that is optimal for the wearable device 100 (such as optimal for the purpose of the wearable device 100).

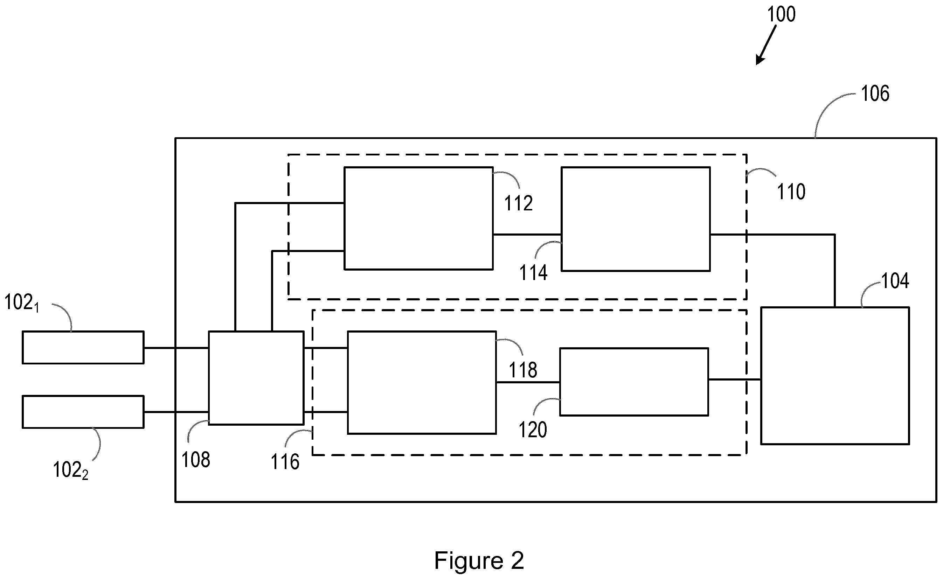

Fig. 2 is a block diagram of a wearable device 100 operable to determine an orientation of the wearable device 100 according to another embodiment. The device 100 is as described earlier with reference to Fig. 1, although only two electrode pairs $102i$, $102_2$ are illustrated and the device 100 is illustrated as comprising further optional components to the plurality of electrode pairs $102i$, $102_2$ and the processor 104. It will be understood, as mentioned above, that the wearable device 100 may comprise any number of multiple electrode pairs and that only two electrode pairs 102i, $102_2$ are illustrated in Fig. 2 as an example.

In the example embodiment illustrated in Fig. 2, the wearable device 100 can also comprise a unit (or module) 106 that comprises the processor 104. The unit 106 may further comprise a switch 108 configured to switch the wearable device 100 between a receiving mode in which the plurality of electrode pairs 102i, $102_2$ are operable as a receiver (or a receiving antenna) and a transmitting mode in which the plurality of electrode pairs $102_1$, $102_2$ are operable as a transmitter (or a transmitting antenna). Thus, as mentioned earlier, the wearable device 100 can be operable as a receiving device or a transmitting device. The unit 106 may also comprise a transmitting part 110 to which the switch connects in the transmitting mode and/or a receiving part 116 to which the switch connects in a receiving mode. Thus, the unit 106 may also be referred to as a transceiver. In some embodiments, the unit 106 may be a radio frequency (RF) unit.

The transmitting part 110 of the unit 106 can, for example, comprise a signal generator 114 that is configured to generate a signal and a voltage booster and driver 112 that is configured to receive the signal generated by the signal generator 114, optionally amplify the signal, and drive at least two electrode pairs 102i, $102_2$ of the plurality of electrode pairs to transmit the signal to a reference device or any other device. The receiving part 116 of the unit 106 can, for example, comprise an analog front end 118 that is configured to receive a signal from at least two electrode pairs 102i, $102_2$ of the plurality of electrode pairs, where the signal is received by the at least two electrode pairs 102i, $102_2$ from a reference device. The receiving part 116 can also comprise an anolog-to-digital converter 120 that is configured to convert the signal from the anaolg front end 118 into a digital signal. The processor 104 can be configured to process the signal from the anolog-to-digital converter 120 of the receiving part 116.

It will be appreciated that Fig. 1 and 2 only show the components required to illustrate certain embodiments and, in a practical implementation, the wearable device 100 may comprise other or additional components to those shown. For example, although not illustrated in Figs. 1 or 2, the wearable device 100 may also comprise a feedback component according to some embodiments. Alternatively or in addition, one or more feedback components may be external to (i.e. separate to or remote from) the wearable device 100. For example, one or more feedback components may be part of another device. A feedback component can be configured to render (or output or provide) an output indicative of whether the wearable device 100 is in the predetermined orientation on the body.

The output can, for example, be a visual output, an audio output, or a tactile (or haptic) output, or any other output, or any combination of outputs. Thus, a feedback component may, for example, comprise a visual feedback component (such as one or more lights, a display, augmented reality glasses, a smart mirror, and/or any other visual feedback component), an audio feedback component (such as one or more speakers and/or any other audio feedback component), and/or a tactile or haptic feedback component (such as one or more vibration actuators or mechanisms and/or any other tactile or haptic feedback component). An example of audio feedback is a spoken instruction (such as 'move wearable device left', 'correct position found', and so on) or a sound (such as a beep), which may vary in amplitude or frequency. An example of visual feedback is arrows on a display, a colour variation (for example, from red to green). An example of tactile or haptic feedback is a vibration, which may vary in intensity and frequency.

Although also not illustrated in Fig. 1 or 2, the wearable device 100 can comprise a memory. Alternatively or in addition, one or more memories may be external to (i.e. separate to or remote from) the wearable device 100. For example, one or more memories may be part of another device. A memory can be configured to store program code that can be executed by the processor 104 of the wearable device 100 to perform the method described herein. Alternatively or in addition, a memory can be configured to store information, data, signals, and measurements acquired or made by the processor 104 of the wearable device 100 or from any components, units, interfaces, sensors, memories, or devices that are external to the wearable device 100. The processor 104 of the wearable device 100 may be configured to control a memory to store information, data, signals, and measurements resulting from the method disclosed herein. For example, the processor 104 of the wearable device 100 may be configured to control a memory to store one or more determined (or current) orientations of the wearable device 100, one or more predetermined orientations for the wearable device 100, or any other information, or any combination of information resulting from the method described herein.

Although also not illustrated in Figs. 1 or 2, in some embodiments, the wearable device 100 may comprise a communications interface (or communications circuitry). The communications interface can be for enabling the wearable device 100 to communicate with (or connect to) one or more other devices, such as the reference device 200, a hub (for example, a router), a smartphone, a tablet, a laptop or any other device, or any combination of devices. Alternatively or in addition, the communications interface can be for enabling the wearable device 100 to communicate with (or connect to) any components, interfaces, units, memories, or sensors, that are internal or external to the wearable device 100. The communications interface may be configured to communicate wirelessly, via a wired connection, or via any other communication (or data transfer) mechanism. In some wireless embodiments, the communications interface may, for example, use radio frequency (RF), Bluetooth, or any other wireless communication technologies, for communications. The wearable device 100 may exchange or transfer data (or information) with one or more other devices, such as any of those mentioned earlier, via the communications interface. In some embodiments, the wearable device 100 may comprise a battery or other power supply for powering the wearable device 100 or means for connecting the wearable device 100 to a mains power supply, or any other component, or any combination of components.

The wearable device 100 described herein may be for any purpose. For example, in some embodiments, the wearable device 100 may comprise a medication dispenser configured to dispense (or deliver or administer) a medication to the body. Alternatively or in addition, in some embodiments, the wearable device 100 may comprise at least one sensor. In embodiments where the wearable device 100 comprises at least one sensor, the wearable device 100 may exchange or transfer data (or information) acquired or measured by the at least one sensor to one or more other devices, such as any of those mentioned earlier (for example, via the communications interface). The at least one sensor can, for example, comprise one or more sensors for monitoring the health of the body. According to some embodiments, the at least one sensor may comprise one or more measurement sensors configured to acquire one or more signals from the body. The signals may, for example, comprise measurement data.

In some embodiments, for example, the wearable device 100 may comprise at least one physiological characteristic (or vital signs) sensor configured to obtain (or acquire or measure) at least one physiological characteristic signal from the body. Examples of a physiological characteristic sensor include, but are not limited to, a heart rate sensor (such as an electrocardiogram ECG sensor) configured to acquire a signal indicative of a heart rate, a heart rate variability sensor configured to acquire a signal indicative of a heart rate variability, a blood pressure sensor configured to acquire a signal indicative of a blood pressure, a skin conductance sensor configured to acquire a signal indicative of a skin conductance response, a skin temperature sensor configured to acquire a signal indicative of a skin temperature, a muscle sensor (such as an electromyography EMG sensor) configured to acquire a signal indicative of muscle activity, or any other physiological characteristic sensor, or any combination of physiological characteristic sensors. Alternatively or in addition, the wearable device 100 may comprise at least one motion sensor configured to acquire motion information from the body. Examples of a motion sensor include, but are not limited to, an accelerometer, a gravity sensor, an inertial sensor, a gyroscope, a magnetometer, or any other motion sensor, or any combination of motion sensors.

Although examples have been provided for the types of sensor that the wearable device 100 may comprise, it will be understood that any other types of sensor or any combinations of sensors are also possible. Also, although examples have been provided for the wearable device 100 comprising a sensor and/or a wearable medication dispenser, it will be understood that the wearable device may have a different purpose and the method disclosed herein can be used in respect of any other type of wearable device. Furthermore, it will be appreciated that the wearable device 100 may comprise additional or alternative components to those described earlier.

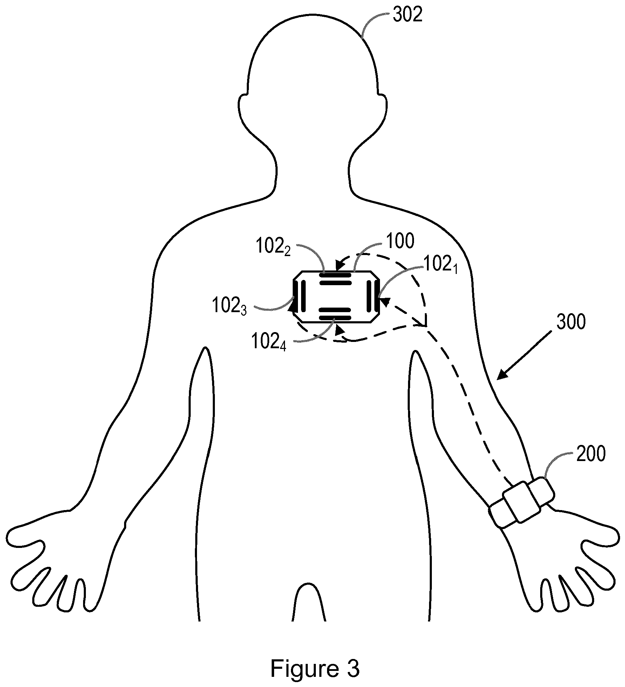

Fig. 3 is an illustration of a system 300 comprising a wearable device 100 operable to determine an orientation of the wearable device 100 according to an embodiment. As illustrated in Fig. 3, the wearable device 100 is configured to be placed on a body 302 and comprises a plurality of electrode pairs $102_1$, $102_2$, $102_3$, $102_4$. Although not illustrated in Fig. 3, the wearable device 100 also comprises a processor 104. More specifically, the wearable device 100 is as described earlier with reference to either of Fig. 1 or 2.

The system 300 of Fig. 3 also comprises a reference device 200. In the illustrated example embodiment of Fig. 3, the reference device 200 is also a wearable device and, more specifically, a wrist-worn device such as a watch or smartwatch. In the system 300 illustrated in Fig. 3, the reference device 200 is operable as the transmitting device and the wearable device 100 is operable as the receiving device. More specifically, the reference device 200 transmits a signal using the body as the transmission medium and at least two electrode pairs $102_1$, $102_2$, $102_3$, $102_4$ of the wearable device 100 receive the signal.

The at least two electrode pairs $102_1$, $102_2$, $102_3$, $102_4$ of the wearable device 100 receive the same signal but at different time intervals due to their location with respect to the reference device 200. More specifically, in the illustrated example system 300 of Fig. 3, the electrode pair $102_1$ first receives the signal, next the electrode pairs $102_2$ and $102_4$ receive the signal (for example, at, or substantially at, the same time) and lastly the electrode pair $102_3$ receives the signal. The electrode pair $102_1$ receives the signal first since it is the electrode pair that is located the closest to (i.e. the least distance from) the reference device 200, whereas the electrode pair $102_3$ receives the signal last since it is the electrode pair that is located the furthest (i.e. the greatest distance) from the reference device 200.

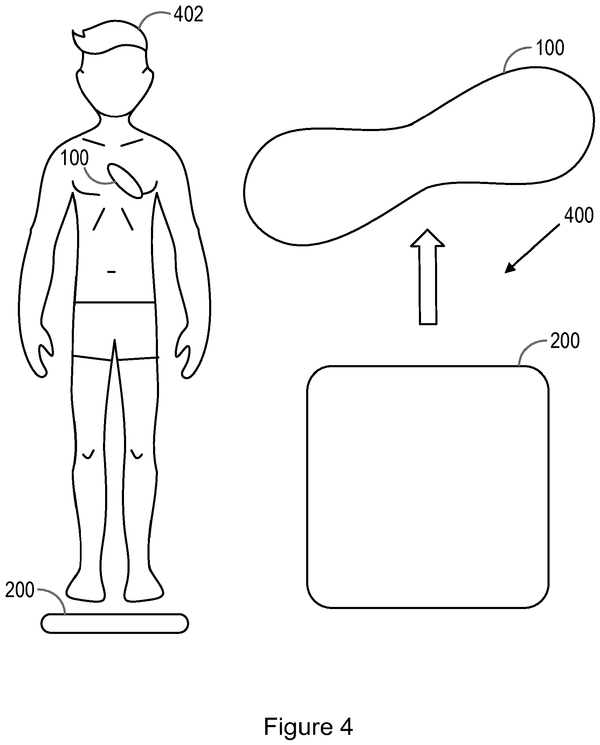

Fig. 4 is an illustration of another system 400 comprising a wearable device 100 operable to determine an orientation of the wearable device 100 according to another embodiment. As illustrated in Fig. 4, the wearable device 100 is configured to be placed on a body 402 and comprises a plurality of electrode pairs $102_1$, $102_2$, $102_3$, $102_4$ (not illustrated in Fig. 4). Although not illustrated in Fig. 4, the wearable device 100 also comprises a processor 104. More specifically, the wearable device 100 is as described earlier with reference to either of Figs. 1 or 2.

The system 400 of Fig. 4 also comprises a reference device 200. In the illustrated example embodiment of Fig. 4, the reference device 200 is a device on which the body 402 can stand, such as a weighing device (or a weighing scales). In the system 400 illustrated in Fig. 4, the wearable device 100 is operable as the transmitting device and the reference device 200 is operable as the receiving device. More specifically, the wearable device 100 transmits a signal from at least two of the plurality of electrode pairs $102_1$, $102_2$, $102_3$, $102_4$ through the body as a transmission medium and the receiver 202 of the reference device 200 receives the signals.

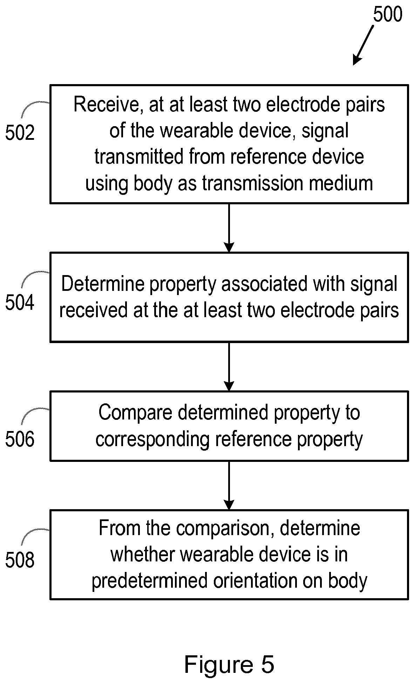

Fig. 5 is a flow chart illustrating a method 500 of operating a wearable device 100 to determine an orientation of the wearable device 100, when placed on a body 302, 402, according to an embodiment. As described earlier, the wearable device 100 comprises a plurality of electrode pairs $102_1$, $102_2$, $102_3$, $102_4$ located at different positions and a processor 104. The illustrated method 500 of Fig. 5 can generally be performed by or under the control of the processor 104 of the wearable device 100. The method 500 will now be described with reference to Figs. 3, 4 and 5.

At block 502 of Fig. 5, the signal transmitted from the reference device 200 is received at at least two of the plurality of electrode pairs $102_1$, $102_2$, $102_3$, $102_4$ using the body 302, 304 as a transmission medium.

Although not illustrated in Fig. 5, in some embodiments, the wearable device 100 may be time synchronised with the reference device 200 prior to the transmission of the signal from the reference device 200. For example, an internal clock (or internal clock generator) of the wearable device 100 and an internal clock (or internal clock generator) of the reference device 200 may be time synchronised prior to the transmission of the signal from the reference device 200. In some embodiments, this synchronisation may be performed by bringing the reference device 200 into proximity of (for example, within a predefined distance of or close to) the wearable device 100, or bringing the wearable device 100 into proximity (for example, within a predefined distance or close to) of the reference device 200 or by connecting the wearable device 100 and the reference device 200 by an electrical connector such as a wire. In some embodiments, the clock signal from the reference device 200 that is operable as the transmitting device may couple to the wearable device 100 that is operable as the receiving device.

As will be described later, the receiver 202 of the reference device 200 may comprise at least one electrode pair. In some embodiments, the internal clock of the reference device 200 may directly couple the clock signal to the wearable device 100 using the electrode pairs of the reference device 200 and the wearable device 100, or the internal clock of the wearable device 100 may directly couple the clock signal to the reference device 200 using the electrode pairs of the wearable device 100 and the reference device 200. In this way, the wearable device 100 may be time synchronised with the reference device 200 or, more specifically, the clock signals of the wearable device 100 and reference device 200 can be time synchronised. In some embodiments, the wearable device 100 may generate a reference signal using the internal synchronised clock of the wearable device 100. The reference signal can provide a reference to the signal transmitted from the reference device 200.

Returning back to Fig. 5, at block 504, a property associated with the signal received at the at least two electrode pairs $102_1$, $102_2$, $102_3$, $102_4$ of the wearable device 100 is determined. More specifically, the property is determined by a processor 104 of the wearable device 100. In some embodiments, a property associated with the signal received at each of the plurality of electrode pairs $102_1$, $102_2$, $102_3$, $102_4$ of the wearable device 100 may be determined. The determined property is indicative of an orientation of the wearable device 100 with respect to the reference device 200.

The determined property associated with the signal received at the at least two electrode pairs $102_1$, $102_2$, $102_3$, $102_4$ of the wearable device 100 can, for example, be indicative of a distance of one of the at least two electrode pairs $102_1$, $102_2$, $102_3$, $102_4$ from the reference device 200 relative to a distance of at least one other of the at least two electrode pairs $102_1$, $102_2$, $102_3$, $102_4$ from the reference device 200. For example, where a determined property associated with the signal received at one of the at least two electrode pairs $102_1$, $102_2$, $102_3$, $102_4$ is the same as a determined property associated with the signal received at the at least one other of the at least two electrode pairs $102_1$, $102_2$, $102_3$, $102_4$, the distance of those electrode pairs $102_1$, $102_2$, $102_3$, $102_4$ from the reference device 200 is the same. In other words, the electrode pairs $102_1$, $102_2$, $102_3$, $102_4$ are the same distance from the reference device 200. On the other hand, where a determined property associated with the signal received at one of the at least two electrode pairs $102_1$, $102_2$, $102_3$, $102_4$ is different to a determined property associated with the signal received at the at least one other of the at least two electrode pairs $102_1$, $102_2$, $102_3$, $102_4$, the distance of those electrode pairs $102_1$, $102_2$, $102_3$, $102_4$ from the reference device 200 is different. In other words, the electrode pairs $102_1$, $102_2$, $102_3$, $102_4$ are a different distance from the reference device 200.

In some embodiments, the property associated with the signal received at the at least two electrode pairs $102_1$, $102_2$, $102_3$, $102_4$ of the wearable device 100 may comprise a phase angle difference between the signal received at the at least two electrode pairs $102_1$, $102_2$, $102_3$, $102_4$ of the wearable device 100. Thus, in these embodiments, the phase angle difference between the signal received at the at least two electrode pairs $102_1$, $102_2$, $102_3$, $102_4$ of the wearable device 100 is indicative of the orientation of the wearable device with respect to the reference device.

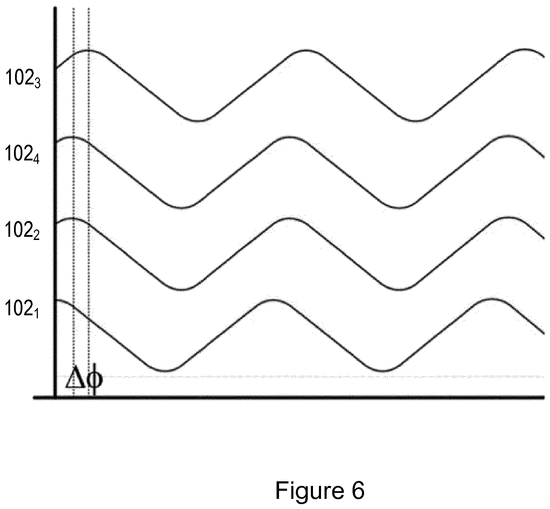

Fig. 6 is an illustration of a phase angle difference between signals received at a plurality of electrodes

$102_1$, $102_2$, $102_3$, $102_4$ of a wearable device 100 according to an embodiment where the wearable device 100 is orientated as shown in Fig. 3. All possible phase angle differences between the signal that is received at the at least two electrode pairs of the wearable device 100 can be described as follows:

$$\emptyset_{ij} = \emptyset_i - \emptyset_j,$$

which indicates the phase angle difference $\emptyset$ between the signal received at electrode pairs $102_i$ and $102_j$ respectively. Accordingly, all phase angle differences $\emptyset_{ii}$ between an electrode pair $102_i$ and itself, where i = 1, 2, 3, 4, is zero. The phase angle difference $\emptyset_{ij}$ between electrode pairs $102_i$ and $102_j$ respectively is the same but the opposite sign to the phase angle difference $\emptyset_{ji}$ between electrode pairs $102_j$ and $102_i$, i.e. $\emptyset_{ij} = -\emptyset_{ji}$, where i = 1, 2, 3, 4 and j = 1, 2, 3, 4 and when i≠j.

For example, $\emptyset_{12} = \emptyset_1 - \emptyset_2$ indicates the phase angle difference between the signal received at the electrode pairs $102_1$ and $102_2$, and so on. Thus, in an embodiment where the wearable device 100 is orientated as shown in Fig. 3, the phase angle difference $\emptyset_{13}$ between the signal received at electrode pairs $102_1$ and $102_3$ respectively will be negative and the phase angle difference $\emptyset_{31}$ between the signal received at electrode pairs $102_3$ and $102_1$ respectively will be positive indicating that electrode pair $102_1$ is closer to the reference device 200 than the electrode pair $102_3$.

The phase angle difference $\emptyset_{24}$ between the signal received at electrode pairs $102_2$ and $102_4$ and the phase angle difference $\emptyset_{42}$ between the signal received at electrode pairs $102_4$ and $102_2$ will be zero (or almost zero) due to an equal (or almost equal) distance between the electrode pairs $102_1$ and $102_4$ and the reference device 200. The phase angle difference $\emptyset_{12}$ between the signal received at electrode pairs $102_1$ and $102_2$ and phase angle difference $\emptyset_{14}$ between the signal received at electrode pairs $102_1$ and $102_4$ will be small but negative and phase angle difference $\emptyset_{32}$ between the signal received at electrode pairs $102_3$ and $102_2$ and the phase angle difference $\emptyset_{34}$ between the signal received at electrode pairs $102_3$ and $102_4$ will be small but positive. These phase angle differences can be used to determine the orientation of the wearable device 100 with respect to the reference device 200.

Alternatively or in addition to the phase angle difference, in some embodiments, the property associated with the signal received at the at least two electrode pairs $102_1$, $102_2$, $102_3$, $102_4$ of the wearable device 100 may comprise a time of flight (ToF) of the signal received at one of the at least two electrode pairs $102_1$, $102_2$, $102_3$, $102_4$ of the wearable device 100 relative to a time of flight of the signal received at at least one other of the at least two electrode pairs $102_1$, $102_2$, $102_3$, $102_4$ of the wearable device 100. Thus, in these embodiments, the relative time of flight of the signals is indicative of the orientation of the wearable device 100 with respect to the reference device 200. More specifically, the longer the time of flight of the signal received at an electrode pair $102_1$, $102_2$, $102_3$, $102_4$ of the wearable device 100, the further away the electrode pair $102_1$, $102_2$, $102_3$, $102_4$ is from the reference device 200. Similarly, the shorter the time of flight of the signal received at an electrode pair $102_1$, $102_2$, $102_3$, $102_4$ of the wearable device 100, the closer the electrode pair $102_1$, $102_2$, $102_3$, $102_4$ is to the reference device 200.

For example, in an embodiment where the wearable device 100 is orientated as shown in Fig. 3, the time of flight $t_1$ from the reference device 200 to the electrode pair $102_1$ is lowest compared to the time of flight $t_2$, $t_3$ and $t_4$ from the reference device 200 to the electrode pairs $102_2$, $102_3$ and $102_4$ respectively. The time of flight $t_3$ from the reference device 200 to the electrode pair $102_3$ has the highest value, while the time of flight $t_2$ and $t_4$ from the reference device 200 to the electrode pairs $102_2$ and $102_4$ respectively will be equal (or almost equal) and less than the time of flight $t_3$ from the reference device 200 to the electrode pair $102_3$ but greater than the time of flight $t_1$ from the reference device 200 to the electrode pair $102_1$.

Thus, where the wearable device 100 is orientated as shown in Fig. 3, the relative time of flight of the signals can be described as follows:

$$t_1 \leq t_2, t_4 \leq t_3.$$

This can provide information on the orientation of the wearable device 100 with respect to the reference device 200. In some embodiments where the property is a time of flight (ToF), the wearable device 100 may be time synchronised with the reference device 200 prior to the transmission of the signal from the reference device 200 (for example, in the manner described earlier). In these embodiments, the wearable device 100 may generate a reference signal using an internal synchronised clock of the wearable device 100. The reference signal can provide a reference to the signal transmitted from the reference device 200.

Alternatively or in addition to the phase angle difference and/or the time of flight, in some embodiments, the property associated with the signal received at the at least two electrode pairs $102_1$, $102_2$, $102_3$, $102_4$ of the wearable device 100 may comprise an amplitude of the signal received at one of the at least two electrode pairs $102_1$, $102_2$, $102_3$, $102_4$ relative to an amplitude of the signal received at at least one other of the at least two electrode pairs $102_1$, $102_2$, $102_3$, $102_4$. Thus, in these embodiments, the relative amplitude of the signals is indicative of the orientation of the wearable device 100 with respect to the reference device 200.

Due to the impedance of the body 302, 402, the signal transmitted from the receiving device 200 undergoes attenuation as it travels through the body 302, 402.

Thus, the amplitude of the signal transmitted from the receiving device 200 decreases as it travels through the body 302, 402. In effect, an amplitude degradation of the signal occurs as the signal travels from the reference device 200 to the wearable device 100. The longer the signal travels through the body 302, 402, the more the signal is attenuated (or the more the amplitude of the signal decreases). Thus, the lower the amplitude of the signal received at an electrode pair $102_1$, $102_2$, $102_3$, $102_4$ of the wearable device 100, the further away the electrode pair $102_1$, $102_2$, $102_3$, $102_4$ is from the reference device 200. Similarly, the higher the amplitude of the signal received at an electrode pair $102_1$, $102_2$, $102_3$, $102_4$ of the wearable device 100, the closer the electrode pair $102_1$, $102_2$, $102_3$, $102_4$ is to the reference device 200.

For example, in an embodiment where the wearable device 100 is orientated as shown in Fig. 3, the amplitude of the signal received at the electrode pair $102_3$ is lowest compared to the amplitude of the signal received at the other electrode pairs $102_1$, $102_2$ and $102_4$. Similarly, the amplitude of the signal received at the electrode pair $102_1$ is highest compared to the amplitude of the signal received at the other electrode pairs $102_2$, $102_3$ and $102_4$. The amplitude of the signal received at the electrode pairs $102_2$ and $102_4$ is equal (or almost equal) and less than the amplitude of the signal received at the electrode pair $102_1$ but greater than the amplitude of the signal received at the electrode pair $102_3$.

In some embodiments, the signal attenuation G may be measured as follows:

$$G = 20 \log_{10} (V_{receive}/V_{send}),$$

where $V_{receive}$ is the amplitude of the signal received at the at least two electrode pairs $102_1$, $102_2$, $102_3$, $102_4$ of the wearable device 100 and $V_{send}$ is the amplitude of the signal transmitted from the reference device 200.

The greater the signal attenuation of the signal received at an electrode pair $102_1$, $102_2$, $102_3$, $102_4$ of the wearable device 100, the further away the electrode pair $102_1$, $102_2$, $102_3$, $102_4$ is from the reference device 200. Similarly, the lesser the attenuation of the signal received at an electrode pair $102_1$, $102_2$, $102_3$, $102_4$ of the wearable device 100, the closer the electrode pair $102_1$, $102_2$, $102_3$, $102_4$ is to the reference device 200. Thus, in an embodiment where the wearable device 100 is orientated as shown in Fig. 3, the attenuation of the signal received at the electrode pair $102_1$ is greater than the attenuation of the signal received at the electrode pair $102_2$ and the attenuation of the signal received at the electrode pair $102_4$ is less than the attenuation of the signal received at the electrode pair $102_3$.

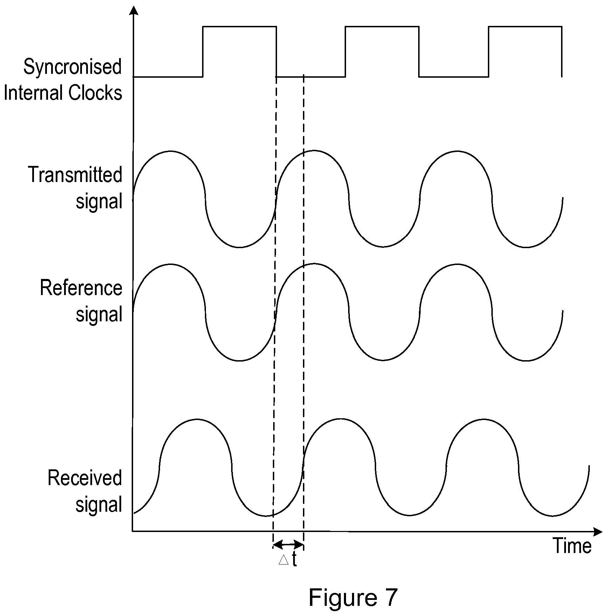

Fig. 7 is an illustration of the signals according to an embodiment where the wearable device 100 is time synchronised with the reference device 200. More specifically, Fig. 7 illustrates a synchronised internal clock signal of the wearable device 100 and the reference device 200, the transmitted signal from the reference device 200 (which operates as the transmitting device in the embodiment described with reference to Fig. 5), the reference signal generated by the wearable device 100 (for example, using the synchronised internal clock signal) and the received signal at the wearable device 100 (which operates as the receiving device in the embodiment described with reference to Fig. 5).

In the embodiment illustrated in Fig. 7, the phase angle difference measured between the reference signal (which provides a reference to the signal transmitted from the reference device 200) and the received signal can provide a value for the time of flight $\Delta t$. For example, one wavelength of a signal (the reference/transmitted/received signal) corresponds to one period of the signal. This means that a phase angle of 360 degrees (for one wavelength) of the signal corresponds to time = 1/operating frequency, where the operating frequency is the operating frequency of the signal. Thus, if the signal is a 100MHz signal, then one period of the signal corresponds to 1/100MHZ, which is 10nS. As such, a phase angle of 360 degrees of the signal corresponds to a time of flight of 10nS and thus a phase angle difference Ø between the signal received at the wearable device 100 with respect to the reference signal corresponds to a time of flight value of Ø*(10/360) ns.

The value of the time of flight $\Delta t$ can provide an indication of the distance between the electrode pairs $102_1$, $102_2$, $102_3$, $102_4$ of the wearable device 100 and reference device 200. If the internal clock of the reference device 200 and the internal clock of the wearable device 100 are time synchronised, then the phase angle difference between the reference signal generated by the wearable device 100 and the received signal at the wearable device 100 can be converted into an amount of time taken for the signal transmitted from the reference device 200 to travel to the electrode pairs $102_1$, $102_2$, $102_3$, $102_4$ of the wearable device 100. The speed C of a signal through a medium (which herein is the body or, more specifically, the skin) is given by $C = f\lambda$, where f is the operating frequency of the transmitted signal and $\lambda$ is the wavelength of the transmitted signal in the medium. Thus, where the time of flight is one full period of the signal, $t = \lambda/C$. As such, the distance d travelled with respect to the time of flight $\Delta t$ is given by $d = \lambda/t^*\Delta t$. The distance d travelled is the distance between the electrode pairs $102_1$, $102_2$, $102_3$, $102_4$ of the wearable device 100 and the reference device 200.

As described earlier, the wearable device 100 comprises a plurality of electrode pairs $102_1$, $102_2$, $102_3$, $102_4$ and the receiver 202 of the reference device 200 may comprise at least one electrode pair. In some embodiments where the receiver 202 of the reference device 200 comprises multiple electrode pairs, each of at least two of the plurality of electrode pairs $102_1$, $102_2$, $102_3$, $102_4$ of the wearable device 100 may be configured to receive a signal transmitted from each electrode pair of the reference device 200 using the body as a transmission medium. In these embodiments, the property associated with a signal received at each of the at least two electrode pairs $102_1$, $102_2$, $102_3$, $102_4$ of the wearable device 100 from each electrode pair of the reference device 200 may be determined. For example, in the embodiment where the property is a relative time of flight, the time of flight of a signal transmitted from each of the electrode pairs of the reference device 200 to each of the electrode pairs $102_1$, $102_2$, $102_3$, $102_4$ of the wearable device 100 may be determined. In this way, additional information can be provided to improve the accuracy of determining the orientation of the wearable device 100 with respect to the reference device 200.

Thus, in the manner described earlier, a property associated with the signal received at the at least two electrode pairs $102_1$, $102_2$, $102_3$, $102_4$ of the wearable device 100 can be determined and, returning back to Fig. 5, at block 506, the determined property is compared to a corresponding reference property. More specifically, the determined property is compared to the corresponding reference property by the processor 104 of the wearable device 100. As mentioned earlier, the determined property is indicative of an orientation of the wearable device 100 with respect to the reference device 200. The corresponding reference property is indicative of a predetermined orientation for the wearable device 100 on the body 302, 402.

At block 508 of Fig. 5, it is determined, from the comparison, whether the wearable device 100 is in the predetermined orientation on the body 302, 402. More specifically, this determination is made by the processor 104 of the wearable device 100. The wearable device 100 can, for example, be determined to be in the predetermined orientation on the body 302, 402 when the determined property matches the corresponding reference property.

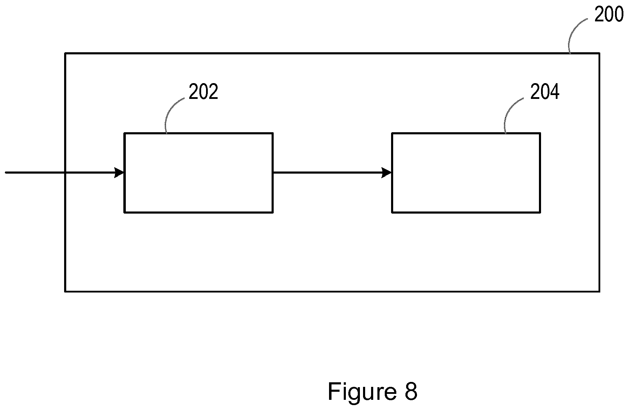

Fig 8 is a block diagram of a reference device 200 operable to determine an orientation of a wearable device 100, when the wearable device 100 is placed on a body, according to an embodiment. The wearable device 100 comprises a plurality of electrode pairs located at different positions

As illustrated in Fig. 8, the reference device 200 comprises a receiver 202 configured to receive signals transmitted from at least two of the plurality of electrode pairs $102_1$, $102_2$, $102_3$, $102_4$ of the wearable device 100 through the body as a transmission medium (or, more specifically, a signal transmission medium). Thus, the signal transmitted from the at least two electrode pairs $102_1$, $102_2$, $102_3$, $102_4$ of the wearable device 100 can be referred to as a body channel sensing (BCS) signal. The signal transmitted can be an electrical signal, such as an alternating current (AC) signal. The signal may be a signal of a certain frequency and amplitude. For example, the signal may be a radio frequency (RF) signal, such as a signal with a frequency in the range of 10 MHz to 150 MHz. As mentioned earlier, this range of frequencies results in the body behaving as a waveguide for signal transmission and enables the determination of the orientation of the wearable device 100 with sufficient accuracy.

In some embodiments, the receiver 202 of the reference device 200 may comprise at least one electrode pair or, more specifically, one electrode pair or multiple electrode pairs. In these embodiments, the at least one electrode pair of the reference device 200 can be configured to receive the signals transmitted from the at least two electrode pairs $102_1$, $102_2$, $102_3$, $102_4$ of the wearable device 100. In some embodiments, the receiver 202 of the reference device 200 (and thus, in some embodiments, the at least one electrode pair of the reference device 200) may be in galvanic contact with the body (or, more specifically, the skin of the body). However, it will be understood that capacitive coupling between the receiver 202 of the reference device 200 (and thus, in some embodiments, the at least one electrode pair of the reference device 200) and the body (or, more specifically, the skin of the body) is sufficient.

As illustrated in Fig. 8, the reference device 200 comprises a processor 204. The processor 204 controls the operation of the reference device 200 and can implement the method in relation to the reference device 200, which is described herein. The processor 204 of the reference device 200 can comprise one or more processors (such as one or more microprocessors MPUs), one or more processing units, one or more multi-core processors and/or one or more controllers (such as one or more microcontrollers MCU), that are configured or programmed to control the reference device 200 in the manner described herein. In particular implementations, the processor 204 of the reference device 200 can comprise a plurality of software and/or hardware modules, each configured to perform, or that are for performing, individual or multiple steps of the method described herein in relation to the reference device 200.

Briefly, the processor 204 of the reference device 200 is configured to determine a property associated with the signals received at the receiver from the at least two electrode pairs $102_1$, $102_2$, $102_3$, $102_4$ and compare the determined property to a corresponding reference property. The determined property is indicative of an orientation of the wearable device 100 with respect to the reference device 200 and the corresponding reference property is indicative of a predetermined orientation for the wearable device 100 on the body. The processor 204 of the reference device 200 is also configured to determine, from the comparison, whether the wearable device 100 is in the predetermined orientation on the body.

It will be appreciated that Fig. 8 only shows the components required to illustrate certain embodiments and, in a practical implementation, the reference device 200 may comprise other or additional components to those shown. For example, although not illustrated in Fig. 8, the reference device 200 may also comprise a feedback component according to some embodiments. Alternatively or in addition, one or more feedback components may be external to (i.e. separate to or remote from) the reference device 200. For example, one or more feedback components may be part of another device. A feedback component can be configured to render (or output or provide) an output indicative of whether the wearable device 100 is in the predetermined orientation on the body. The output can, for example, be a visual output, an audio output, or a tactile output, or any other output, or any combination of outputs. Thus, a feedback component may, for example, comprise a visual feedback component (such as one or more lights), an audio feedback component (such as one or more speakers), and/or a tactile feedback component (such as one or more vibration actuators or mechanisms).

Although also not illustrated in Fig. 8, the reference device 200 can comprise a memory. Alternatively or in addition, one or more memories may be external to (i.e. separate to or remote from) the reference device 200. For example, one or more memories may be part of another device. A memory can be configured to store program code that can be executed by the processor 204 of the reference device 200 to perform the method described herein. Alternatively or in addition, a memory can be configured to store information, data, signals, and measurements acquired or made by the processor 204 of the reference device 200 or from any components, units, interfaces, sensors, memories, or devices that are external to the reference device 200. The processor 204 of the reference device 200 may be configured to control a memory to store information, data, signals, and measurements resulting from the method disclosed herein. For example, the processor 204 of the reference device 200 may be configured to control a memory to store one or more determined (or current) orientations of the wearable device 100, one or more predetermined orientations for the wearable device 100, or any other information, or any combination of information resulting from the method described herein.

Although also not illustrated in Fig. 8, in some embodiments, the reference device 200 may comprise a communications interface (or communications circuitry). The communications interface can be for enabling the reference device 200 to communicate with (or connect to) one or more other devices, such as the wearable device 100, a hub (for example, a router), a smartphone, a tablet, a laptop or any other device, or any combination of devices. Alternatively or in addition, the communications interface can be for enabling the reference device 200 to communicate with (or connect to) any components, interfaces, units, memories, or sensors, that are internal or external to the reference device 200. The communications interface may be configured to communicate wirelessly, via a wired connection, or via any other communication (or data transfer) mechanism. In some wireless embodiments, the communications interface may, for example, use radio frequency (RF), Bluetooth, or any other wireless communication technologies, for communications. The reference device 200 may exchange or transfer data (or information) with one or more other devices, such as any of those mentioned earlier, via the communications interface. In some embodiments, the reference device 200 may comprise a battery or other power supply for powering the reference device 200 or means for connecting the reference device 200 to a mains power supply, or any other component, or any combination of components.

Although examples have been provided, it will be appreciated that the reference device 200 may comprise additional or alternative components to those described earlier.

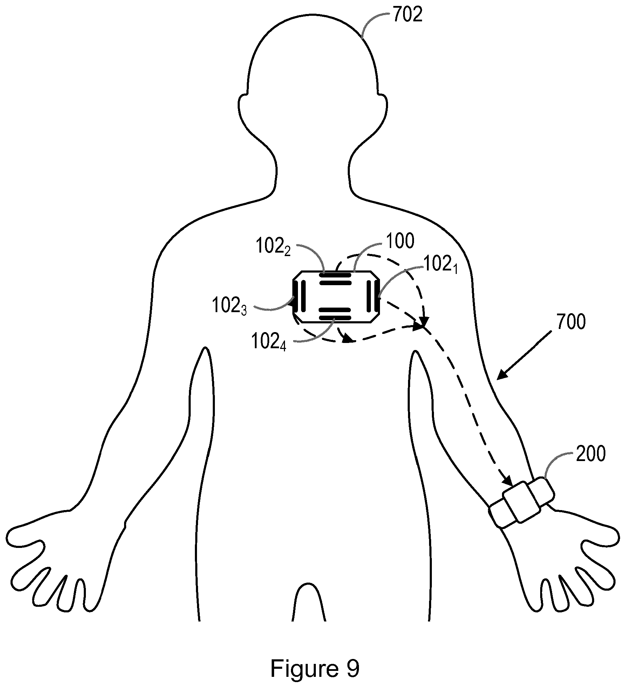

Fig. 9 is an illustration of a system 700 comprising a reference device 200 operable to determine an orientation of a wearable device 100 according to an embodiment. As illustrated in Fig. 9, the reference device 200 is configured to be placed on a body 702. Although not illustrated in Fig. 9, the reference device 200 comprises a receiver 202 and a processor 204. More specifically, the reference device 200 is as described earlier with reference to Fig. 8. In the illustrated example embodiment of Fig. 9, the reference device 200 is a wearable device and, more specifically, a wrist-worn device such as a watch or smartwatch.

The system 700 of Fig. 9 also comprises a wearable device 100, which is configured to be placed on the body 302. The wearable device 100 comprises a plurality of electrode pairs $102_1$, $102_2$, $102_3$, $102_4$. In the system 700 illustrated in Fig. 9, the wearable device 100 is operable as the transmitting device and the reference device 200 is operable as the receiving device. More specifically, at least two electrode pairs $102_1$, $102_2$, $102_3$, $102_4$ of the wearable device 100 transmit a signal using the body as the transmission medium and the receiver 202 of the reference device 200 receives the signals.

The receiver 202 of the reference device 200 receives the signals at different time intervals due to the location of the at least two electrode pairs $102_1$, $102_2$, $102_3$, $102_4$ of the wearable device 100 with respect to the reference device 200. More specifically, in the illustrated example system 700 of Fig. 9, the receiver 202 of the reference device 200 first receives the signal from the electrode pair $102_1$, next receives the signal from the electrode pairs $102_2$ and $102_4$ (for example, at or substantially at the same time) and lastly receives the signal from the electrode pair $102_3$. The receiver 202 of the reference device 200 receives the signal from the electrode pair $102_1$ first since the electrode pair $102_1$ is the electrode pair that is located the closest to (i.e. the least distance from) the reference device 200, whereas receiver 202 of the reference device 200 receives the signal from the electrode pair $102_3$ last since the electrode pair $102_3$ is the electrode pair that is located the furthest (i.e. the greatest distance) from the reference device 200.

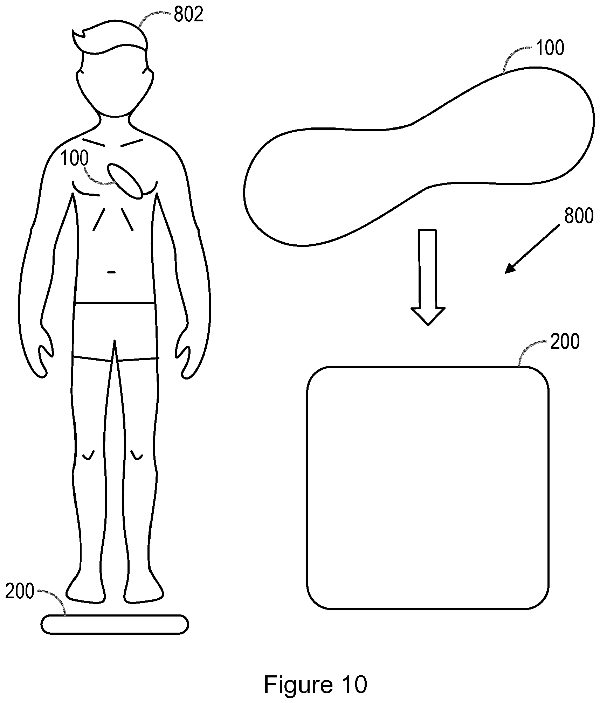

Fig. 10 is an illustration of another system 800 comprising a reference device 200 operable to determine an orientation of a wearable device 100 according to another embodiment. As illustrated in Fig. 10, the reference device 200 is contactable with at least part of a body 802.

In the illustrated example embodiment of Fig. 10, the reference device 200 is a device on which the body 802 can stand, such as a weighing device (or a weighing scales). Although not illustrated in Fig. 10, the reference device 200 also comprises a processor 204. More specifically, the reference device 200 is as described earlier with reference to Fig. 8.

The system 800 of Fig. 10 also comprises a wearable device 100. The wearable device 100 is configured to be placed on the body 802 and comprises a plurality of electrode pairs $102_1$, $102_2$, $102_3$, $102_4$ (not illustrated in Fig. 10). In the system 800 illustrated in Fig. 10, the wearable device 100 is operable as the transmitting device and the reference device 200 is operable as the receiving device. More specifically, the wearable device 100 transmits a signal from at least two of the plurality of electrode pairs $102_1$, $102_2$, $102_3$, $102_4$ through the body as a transmission medium and the receiver 202 of the reference device 200 receives the signals.

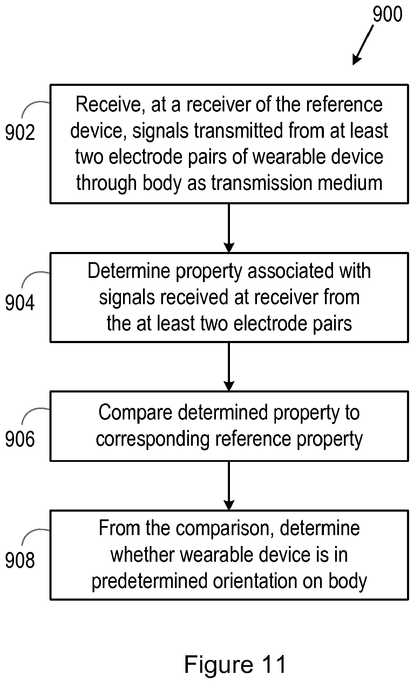

Fig. 11 is a flow chart illustrating a method 900 of operating a reference device 200 to determine an orientation of the wearable device 100, when the wearable device 100 is placed on a body 702, 802 according to an embodiment. As described earlier, the wearable device 100 comprises a plurality of electrode pairs $102_1$, $102_2$, $102_3$, $102_4$ located at different positions. The illustrated method 900 of Fig. 11 can generally be performed by or under the control of the processor 204 of the reference device 200. The method 900 will now be described with reference to Figs. 9, 10 and 11.

At block 902 of Fig. 11, signals transmitted from at least two of the plurality of electrode pairs $102_1$, $102_2$, $102_3$, $102_4$ of the wearable device 100 through the body 702, 802 as a transmission medium are received at the receiver 202 of the reference device 200. In some embodiments, the signals may be sequentially transmitted from the at least two electrode pairs $102_1$, $102_2$, $102_3$, $102_4$ of the wearable device 100. The reference device 200 may be notified as to which of the at least two electrode pairs $102_1$, $102_2$, $102_3$, $102_4$ of the wearable device 100 is currently transmitting a signal. For example, a protocol may be used to indicate to the reference device 200 which of the at least two electrode pairs $102_1$, $102_2$, $102_3$, $102_4$ of the wearable device 100 is currently transmitting a signal. In this way, each time a signal is transmitted from an electrode pair $102_1$, $102_2$, $102_3$, $102_4$ of the wearable device 100, the reference device 200 knows which electrode pair $102_1$, $102_2$, $102_3$, $102_4$ transmitted the signal that is received by the receiver 202 of the reference device 200.

Although not illustrated in Fig. 11, in some embodiments, the reference device 200 may be time synchronised with the wearable device 100 prior to the transmission of the signals from the at least two electrode pairs $102_1$, $102_2$, $102_3$, $102_4$ of the wearable device 100. For example, an internal clock (or internal clock generator) of the wearable device 100 and an internal clock (or internal clock generator) of the reference device 200 may be time synchronised prior to the transmission of the signal from the at least two electrode pairs $102_1$, $102_2$, $102_3$, $102_4$ of the wearable device 100. In some embodiments, this synchronisation may be performed by bringing the reference device 200 into proximity of (for example, within a predefined distance of or close to) the wearable device 100, or bringing the wearable device 100 into proximity (for example, within a predefined distance or close to) of the reference device 200 or by connecting the wearable device 100 and the reference device 200 by an electrical connector such as a wire. In some embodiments, the clock signal from the wearable device 100 that is operable as the transmitting device may couple to the reference device 200 that is operable as the receiving device.

As described earlier, the receiver 202 of the reference device 200 may comprise at least one electrode pair. In some embodiments, the internal clock of the reference device 200 may directly couple the clock signal to the wearable device 100 using the electrode pairs of the reference device 200 and the wearable device 100, or the internal clock of the wearable device 100 may directly couple the clock signal to the reference device 200 using the electrode pairs of the wearable device 100 and the reference device 200. In this way, the wearable device 100 may be time synchronised with the reference device 200 or, more specifically, the clock signals of the wearable device 100 and reference device 200 can be time synchronised. In some embodiments, the reference device 200 may generate a reference signal using the internal synchronised clock of the reference device 200.

Returning back to Fig. 11, at block 904, a property associated with the signals received at the receiver 202 of the reference device 200 from the at least two electrode pairs $102_1$, $102_2$, $102_3$, $102_4$ of the wearable device 100 is determined. More specifically, the property is determined by a processor 204 of the reference device 200. In some embodiments, a property associated with the signal received at the receiver 202 of the reference device 200 from each of the plurality of electrode pairs $102_1$, $102_2$, $102_3$, $102_4$ of the wearable device 100 may be determined. The determined property is indicative of an orientation of the wearable device 100 with respect to the reference device 200.

The determined property associated with the signal received at the receiver 202 of the reference device 200 can, for example, be indicative of a distance of one of the at least two electrode pairs $102_1$, $102_2$, $102_3$, $102_4$ from the reference device 200 relative to a distance of at least one other of the at least two electrode pairs $102_1$, $102_2$, $102_3$, $102_4$ from the reference device 200. For example, where a determined property associated with the signal received at the receiver 202 of the reference device 200 from one of the at least two electrode pairs $102_1$, $102_2$, $102_3$, $102_4$ is the same as a determined property associated with the signal received at the receiver 202 of the reference device 200 from at least one other of the at least two electrode pairs $102_1$, $102_2$, $102_3$, $102_4$, the

distance of those electrode pairs $102_1$, $102_2$, $102_3$, $102_4$ from the reference device 200 is the same. In other words, the electrode pairs $102_1$, $102_2$, $102_3$, $102_4$ are the same distance from the reference device 200. On the other hand, where a determined property associated with the signal received at the receiver 202 of the reference device 200 from one of the at least two electrode pairs $102_1$, $102_2$, $102_3$, $102_4$ is different to a determined property associated with the signal received at the receiver 202 of the reference device 200 from at least one other of the at least two electrode pairs $102_1$, $102_2$, $102_3$, $102_4$, the distance of those electrode pairs $102_1$, $102_2$, $102_3$, $102_4$ from the reference device 200 is different. In other words, the electrode pairs $102_1$, $102_2$, $102_3$, $102_4$ are a different distance from the reference device 200.

In some embodiments, the property associated with the signals received at the receiver 202 of the reference device 200 from the at least two electrode pairs $102_1$, $102_2$, $102_3$, $102_4$ of the wearable device 100 may comprise a phase angle difference between the signals received at the receiver 202 of the reference device 200 from the at least two electrode pairs $102_1$, $102_2$, $102_3$, $102_4$ of the wearable device 100. Thus, in these embodiments, the phase angle difference between the signals received at the receiver 202 of the reference device 200 is indicative of the orientation of the wearable device with respect to the reference device.

The phase angle difference between signals received at the receiver 202 of the receiving device 200 from the at least two electrode pairs $102_1$, $102_2$, $102_3$, $102_4$ of the wearable device 100, where the wearable device 100 is orientated as shown in Fig. 9, is as shown in Fig. 6. All possible phase angle differences between the signal that is received at the receiver 202 of the reference device 200 from the at least two electrode pairs can be described as follows:

$$\emptyset_{ij} = \emptyset_i - \emptyset_j,$$

which indicates the phase angle difference Ø between the signal received at the receiver 202 of the reference device 200 from the at least two electrode pairs $102_i$ and $102_j$ respectively. Accordingly, all phase angle differences $\emptyset_{ii}$ between an electrode pair $102_i$ and itself, where i = 1, 2, 3, 4, is zero. The phase angle difference $\emptyset_{ij}$ between electrode pairs $102_i$ and $102_j$ respectively is the same but the opposite sign to the phase angle difference $\emptyset_{ji}$ between electrode pairs $102_j$ and $102_i$, i.e. $\emptyset_{ij} = - \emptyset_{ji}$, where i = 1, 2, 3, 4.

For example, $\emptyset_{12} = \emptyset_1 - \emptyset_2$ indicates the phase angle difference between the signal received at the receiver 202 of the reference device 200 from the electrode pairs $102_1$ and $102_2$, and so on. Thus, where the wearable device 100 is orientated as shown in Fig. 9, the phase angle difference $\emptyset_{13}$ between the signal received at the receiver 202 of the reference device 200 from electrode pairs $102_1$ and $102_3$ respectively will be negative and the phase angle difference $\emptyset_{31}$ between the signal received at the receiver 202 of the reference device 200 from electrode pairs $102_3$ and $102_1$ respectively will be positive indicating that electrode pair $102_1$ is closer to the reference device 200 than the electrode pair $102_3$.

The phase angle difference $\emptyset_{24}$ between the signal received at the receiver 202 of the reference device 200 from electrode pairs $102_2$ and $102_4$ and the phase angle difference $\emptyset_{42}$ between the signal received at the receiver 202 of the reference device 200 from electrode pairs $102_4$ and $102_2$ will be zero (or almost zero) due to an equal (or almost equal) distance between the electrode pairs $102_1$ and $102_4$ and the reference device 200. The phase angle difference $\emptyset_{12}$ between the signal received at the receiver 202 of the reference device 200 from electrode pairs $102_1$ and $102_2$ and phase angle difference $\emptyset_{14}$ between the signal received at the receiver 202 of the reference device 200 from electrode pairs $102_1$ and $102_4$ will be small but negative and phase angle difference $\emptyset_{32}$ between the signal received at the receiver 202 of the reference device 200 from electrode pairs $102_3$ and $102_2$ and the phase angle difference $\emptyset_{34}$ between the signal received at the receiver 202 of the reference device 200 from electrode pairs $102_3$ and $102_4$ will be small but positive. These phase angle differences can be used to determine the orientation of the wearable device 100 with respect to the reference device 200.

Alternatively or in addition, in some embodiments, the property associated with the signals received at the receiver 202 of the reference device 200 from the at least two electrode pairs $102_1$, $102_2$, $102_3$, $102_4$ of the wearable device 100 may comprise a time of flight of the signal received at the receiver 202 of the reference device 200 from one of the at least two electrode pairs $102_1$, $102_2$, $102_3$, $102_4$ of the wearable device 100 relative to a time of flight of the signal received at the receiver from at least one other of the at least two electrode pairs $102_1$, $102_2$, $102_3$, $102_4$ of the wearable device 100. Thus, in these embodiments, the relative time of flight signal is indicative of the orientation of the wearable device 100 with respect to the reference device 200.

For example, where the wearable device 100 is orientated as shown in Fig. 9, the time of flight $t_1$ from the electrode pair $102_1$ to the reference device 200 is lowest compared to the time of flight $t_2$, $t_3$ and $t_4$ from the electrode pairs $102_2$, $102_3$ and $102_4$ respectively to the reference device 200. The time of flight $t_3$ from the electrode pair $102_3$ to the reference device 200 has the highest value, while the time of flight $t_2$ and $t_4$ from the electrode pairs $102_2$ and $102_4$ respectively to the reference device 200 will be equal (or almost equal) and less than the time of flight $t_3$ from the electrode pair $102_3$ to the reference device 200 but greater than the time of flight $t_1$ from the electrode pair $102_1$ to the reference device 200.

Thus, where the wearable device 100 is orientated as shown in Fig. 9, the relative time of flight of the signals can be described as follows:

$$t_1 \leq t_2, \; t_4 \leq t_3.$$

This can provide information on the orientation of the wearable device 100 with respect to the reference device 200. In some embodiments where the property is a time of flight (ToF), the wearable device 100 may be time synchronised with the reference device 200 prior to the transmission of the signal from the reference device 200 (for example, in the manner described earlier). In these embodiments, the wearable device 100 may generate a reference signal using an internal synchronised clock of the wearable device 100. As described earlier, the signals according to an embodiment where the wearable device 100 is time synchronised with the reference device 200 is provided with reference to Fig. 7. However, in the embodiment described with reference to Fig. 11, the transmitted signal is from the wearable device 100 (which operates as the transmitting device in the embodiment described with reference to Fig. 11), the reference signal is generated by the reference device 200 (for example, using the synchronised internal clock signal) and the received signal at the reference device 200 (which operates as the receiving device in the embodiment described with reference to Fig. 11).

Alternatively or in addition to the phase angle difference and/or the time of flight, in some embodiments, the property associated with the signal received at the receiver 202 of the reference device 200 from the at least two electrode pairs $102_1$, $102_2$, $102_3$, $102_4$ of the wearable device 100 may comprise an amplitude of the signal received at the receiver 202 from one of the at least two electrode pairs $102_1$, $102_2$, $102_3$, $102_4$ relative to an amplitude of the signal received at the receiver 200 from at least one other of the at least two electrode pairs. Thus, in these embodiments, the relative amplitude of the signals is indicative of the orientation of the wearable device 100 with respect to the reference device 200.

Due to the impedance of the body 702, 802, the signal transmitted from the at least two electrode pairs $102_1$, $102_2$, $102_3$, $102_4$ of the wearable device 100 undergoes attenuation as it travels through the body 702, 802. Thus, the amplitude of the signal transmitted from the at least two electrode pairs $102_1$, $102_2$, $102_3$, $102_4$ of the wearable device 100 decreases as it travels through the body 702, 802. In effect, an amplitude degradation of the signal occurs as the signal travels from the at least two electrode pairs $102_1$, $102_2$, $102_3$, $102_4$ of the wearable device 100 to the reference device 200. The longer the signal travels through the body 702, 802, the more the signal is attenuated (or the more the amplitude of the signal decreases). Thus, the lower the amplitude of the signal received at the reference device 200, the further away the electrode pair $102_1$, $102_2$, $102_3$, $102_4$ that transmitted the signal is from the reference device 200. Similarly, the higher the amplitude of the signal received at the reference device 200, the closer the electrode pair $102_1$, $102_2$, $102_3$, $102_4$ that transmitted the

signal is to the reference device 200.

For example, in an embodiment where the wearable device 100 is orientated as shown in Fig. 9, the amplitude of the signal received at the receiver 202 of the reference device 200 from the electrode pair $102_3$ is lowest compared to the amplitude of the signal received at the receiver 202 of the reference device 200 from the other electrode pairs $102_1$, $102_2$ and $102_4$. Similarly, the amplitude of the signal received at the receiver 202 of the receiving device 200 from the electrode pair $102_1$ is highest compared to the amplitude of the signal received at the receiver 202 of the receiving device 200 from the other electrode pairs $102_2$, $103_3$ and $102_4$. The amplitude of the signal received at the receiver 202 of the reference device 200 from the electrode pairs $102_2$ and $102_4$ is equal (or almost equal) and less than the amplitude of the signal received at the receiver 202 of the reference device 200 from the electrode pair $102_1$ but greater than the amplitude of the signal received at the receiver 202 of the reference device 200 from the electrode pair $102_3$.

In some embodiments, the signal attenuation G may be measured as follows:

$$G = 20\log_{10}\left(V_{receive}/V_{send}\right),$$

where $V_{receive}$ is the amplitude of the signal received at the receiver 202 of the reference device 200 from the at least two electrode pairs $102_1$, $102_2$, $102_3$, $102_4$ of the wearable device 100 and $V_{send}$ is the amplitude of the signal transmitted from the at least two electrode pairs $102_1$, $102_2$, $102_3$, $102_4$ of the wearable device 100. The greater the signal attenuation of the signal received at the receiver 202 of the reference device 200 from an electrode pair $102_1$, $102_2$, $102_3$, $102_4$ of the wearable device 100, the further away the electrode pair $102_1$, $102_2$, $102_3$, $102_4$ is from the reference device 200. Similarly, the lesser the attenuation of the signal received at the receiver 202 of the reference device 200 from an electrode pair $102_1$, $102_2$, $102_3$, $102_4$ of the wearable device 100, the closer the electrode pair $102_1$, $102_2$, $102_3$, $102_4$ is to the reference device 200. Thus, in an embodiment where the wearable device 100 is orientated as shown in Fig. 9, the attenuation of the signal received at the receiver 202 of the reference device 200 from the electrode pair $102_1$ is greater than the attenuation of the signal received at the receiver 202 of the reference device 200 from the electrode pair $102_2$ and the attenuation of the signal received at the receiver 202 of the reference device 200 from the electrode pair $102_4$ is less than the attenuation of the signal received at the receiver 202 of the reference device 200 from the electrode pair $102_3$.

As described earlier, the wearable device 100 comprises a plurality of electrode pairs $102_1$, $102_2$, $102_3$, $102_4$ and the receiver 202 of the reference device 200 may comprise at least one electrode pair. In some embodiments where the receiver 202 of the reference device 200 comprises multiple electrode pairs, each of the electrode pairs of the reference device 200 may be configured to receive a signal transmitted from each of at least two of the plurality of electrode pairs $102_1$, $102_2$, $102_3$, $102_4$ of the wearable device 100 through the body as a transmission medium. In these embodiments, the property associated with a signal received at each of the electrode pair of the reference device 200 from each of the at least two electrode pairs $102_1$, $102_2$, $102_3$, $102_4$ of the wearable device 100 may be determined. For example, in the embodiment where the property is a relative time of flight, the time of flight of a signal transmitted from each of the electrode pairs $102_1$, $102_2$, $102_3$, $102_4$ of the wearable device 100 to each of the electrode pairs of the reference device 200 may be determined. In this way, additional information can be provided to improve the accuracy of determining the orientation of the wearable device 100 with respect to the reference device 200.

Thus, in the manner described earlier, a property associated with the signals received at the receiver 202 of the reference device 200 from the at least two electrode pairs $102_1$, $102_2$, $102_3$, $102_4$ of the wearable device 100 can be determined and, returning back to Fig. 11, at block 906, the determined property is compared to a corresponding reference property. More specifically, this comparison is performed by the processor 204 of the reference device 200. As mentioned earlier, the determined property is indicative of an orientation of the wearable device 100 with respect to the reference device 200. The corresponding reference property is indicative of a predetermined orientation for the wearable device 100 on the body 702, 802.

At block 908 of Fig. 11, it is determined, from the comparison, whether the wearable device 100 is in the predetermined orientation on the body 302, 402. More specifically, this determination is made by the processor 104 of the wearable device 100. The wearable device 100 can, for example, be determined to be in the predetermined orientation on the body 302, 402 when the determined property matches the corresponding reference property.

Although not illustrated in Fig. 5 or 11, in some embodiments, either of the methods 500, 900 may further comprise controlling a feedback component to render (or output or provide) an output indicative of whether the wearable device 100 is in the predetermined orientation on the body 302, 402, 702, 802. For example, the output may provide an indication that informs a user to stop orientating the wearable device 100 when the predetermined orientation is reached (or achieved). More specifically, the processor 102 of the wearable device 100 may control the feedback component in this manner in the embodiment described with reference to Fig. 5 and the processor 202 of the wearable device 200 may control the feedback component in this manner in the embodiment described with reference to Fig. 11. As described earlier, the output can be a visual output, an audio output, or a tactile output, or any other output, or any combination of outputs.

In an example, a user of the wearable device 100 may randomly orientate (for example, randomly rotate) the wearable device 100 until the wearable device 100 is in the predetermined orientation on the body 302, 402, 702, 802, where the user is notified by the feedback component that the predetermined orientation is reached. For example, when the predetermined orientation is reached, the visual feedback component may output a light, an audio feedback component may output a sound, or a tactile feedback component may output a vibration. The user may secure (for example, attach, stick, or adhere) the wearable device 100 in place on the body at the predetermined orientation once the user is notified that the predetermined orientation is reached.

In the systems 300, 400 illustrated in Figs. 3 and 4, the wearable device 100 is configured in the manner described earlier with reference to Fig. 1 or 2 and operable in the manner described earlier with reference to Fig. 5 to determine the orientation of the wearable device 100. More generally, in these embodiments, the reference device 200 is operable as a transmitting device and the wearable device 100 is operable as a receiving device. Thus, according to these embodiments, the wearable device 100 (rather than the reference device 200) has the capabilities to determine the orientation of the wearable device 100. In these embodiments, the reference device 200 can be any general reference device 200 configured to transmit the signal to the at least two electrodes of the wearable device 100.

On the other hand, in the systems 700, 800 illustrated in Figs. 9 and 10, the reference device 200 is configured in the manner described earlier with reference to Fig. 8 and operable in the manner described earlier with reference to Fig. 11 to determine the orientation of the wearable device 100. More generally, in these embodiments, the wearable device 100 is operable as a transmitting device and the reference device 200 is operable as a receiving device. Thus, according to these embodiments, the reference device 200 (rather than the wearable device 100) has the capabilities to determine the orientation of the wearable device 100 according to these other embodiments. In these embodiments, the wearable device 100 can be any general wearable device comprising the at least two electrodes configured to transmit the signals to the reference device 200.

In yet further embodiments, the wearable device 100 may be configured in the manner described earlier with reference to Fig. 1 or 2 and operable in the manner described earlier with reference to Fig. 5 to determine the orientation of the wearable device 100 and the reference device 200 may be configured in the manner described earlier with reference to Fig. 8 and operable in the manner described earlier with reference to Fig. 11 to determine the orientation of the wearable device 100. More generally, in these embodiments, the reference device 200 may be operable as a transmitting device when the wearable device 100 is operable as a receiving device and the wearable device 100 may be operable as a transmitting device when the reference device 200 is operable as a receiving device. Thus, according to these embodiments, both the wearable device 100 and the reference device 200 may have the capabilities necessary to determine the orientation of the wearable device 100.

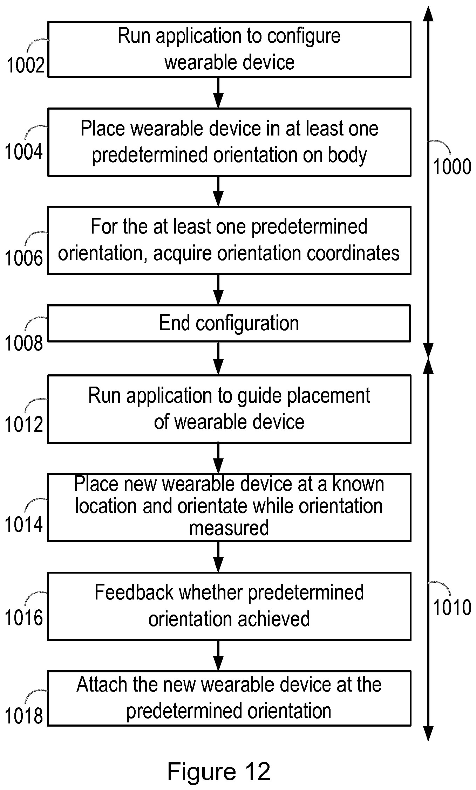

Fig. 12 is a flow chart illustrating the method of operating the wearable device 100 or the reference device 200 to determine the orientation of the wearable device 100 in use, when the wearable device 100 is placed on a body 302, 402, 702, 802, according to an example embodiment. The method comprises a calibration phase 1000 and a device placement phase 1010.

At block 1002 of Fig. 12, an application is run to configure a wearable device 100. For example, a user of the wearable device 100 may input an instruction to the wearable device 100 to run the application to configure the wearable device 100. The user may be a trained user such as a caregiver, a medical person, a clinician, or any other user trained in wearable device 100 placement. At block 1004 of Fig. 12, the user of the wearable device 100 places the wearable device 100 in at least one predetermined orientation on a body. At block 1006 of Fig. 12, for the at least one predetermined orientation, orientation coordinates of the wearable device 100 are acquired. The orientation coordinates of the wearable device 100 may also be recorded or stored on the wearable device 100, on the reference device 200, or on another device. At block 1008 of Fig. 12, the configuration phase ends. For example, the user of the wearable device 100 may input an instruction to the wearable device 100 to end the configuration phase.

At block 1012 of Fig. 12, an application is run to guide placement of wearable device. For example, a further user of the wearable device 100 may input an instruction to the wearable device 100 to run the application to guide placement of the wearable device 100. The further user may be an untrained user such as a subject, a patient, or any other user untrained in wearable device 100 placement. From block 1012 onwards, the further user can properly place (or replace) a wearable device 100 themselves by running the application on the wearable device 100, which guides the further user to correctly place (or replace) the wearable device 100. At block 1014 of Fig. 12, a new wearable device 100 is paced by the further user at a known location on a body and orientated. While the wearable device 100 is orientated, the orientation of the wearable device 100 is determined (or measured) in the manner described earlier with reference to Fig. 5 or 11. The orientation of the wearable device 100 may be determined (or measured) continuously.

At block 1016 of Fig. 12, a feedback component is controlled to render (or output or provide) an output indicative of whether the wearable device 100 is in the predetermined orientation on the body 302, 402, 702, 802. For example, as described earlier, the output may provide an indication that informs a user to stop orientating the wearable device 100 when the predetermined orientation is reached (or achieved). At block 1018 of Fig. 12, the new wearable device 100 is placed on (and, for example, attached to) the body by the further user at the predetermined orientation.

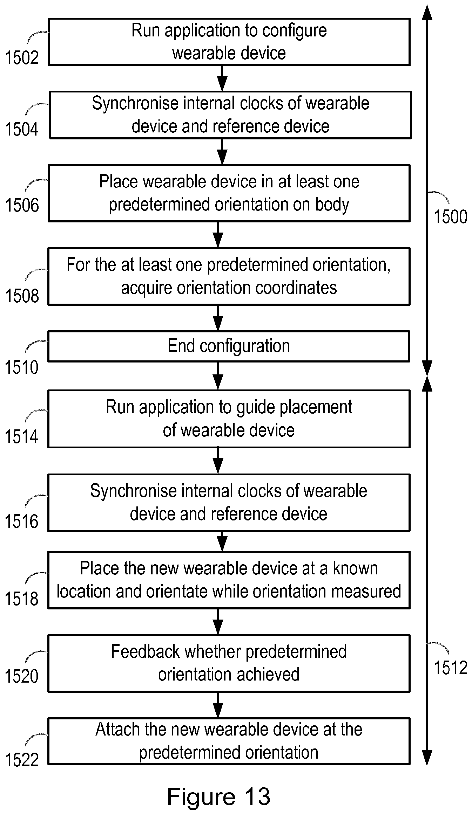

Fig. 13 is a flow chart illustrating the method of operating the wearable device 100 or the reference device 200 to determine the orientation of the wearable device 100 in use, when the wearable device 100 is placed on a body 302, 402, 702, 802, according to another example embodiment. The method comprises a calibration phase 1500 and a device placement phase 1512.

At block 1502 of Fig. 13, an application is run to configure a wearable device 100. For example, a user of the wearable device 100 may input an instruction to the wearable device 100 to run the application to configure the wearable device 100. The user may be a trained user such as a caregiver, a medical person, a clinician, or any other user trained in wearable device 100 placement. At block 1504 of Fig. 13, the internal clocks of the wearable device 100 and the reference device 200 are time synchronised, for example, in the manner described earlier. For example, the wearable device 100 may be brought into close proximity to the reference device 200 by the user or the wearable device 100 and the reference device 200 may be connected by an electrical connector (such as a wire) to synchronise the internal clocks of wearable device 100 and the reference device 200.

At block 1506 of Fig. 13, the user of the wearable device 100 places the wearable device 100 in at least one predetermined orientation on a body. At block 1508 of Fig. 13, for the at least one predetermined orientation, orientation coordinates of the wearable device 100 are acquired. The orientation coordinates of the wearable device 100 may also be recorded or stored on the wearable device 100, on the reference device 200, or on another device. At block 1510 of Fig. 13, the configuration phase ends. For example, the user of the wearable device 100 may input an instruction to the wearable device 100 to end the configuration phase.

At block 1514 of Fig. 13, an application is run to guide placement of wearable device. For example, a further user of the wearable device 100 may input an instruction to the wearable device 100 to run the application to guide placement of the wearable device 100. The further user may be an untrained user such as a subject, a patient, or any other user untrained in wearable device 100 placement. From block 1514 onwards, the further user can properly place (or replace) a wearable device 100 themselves by running the application on the wearable device 100, which guides the further user to correctly place (or replace) the wearable device 100.

At block 1516 of Fig. 13, the internal clocks of a new wearable device 100 and the reference device 200 are time synchronised, for example, in the manner described earlier. For example, the new wearable device 100 may be brought into close proximity to (for example, placed on) the reference device 200 or the new wearable device 100 and the reference device 200 may be connected by an electrical connector (such as a wire) by the further user to synchronise the internal clocks of the new wearable device 100 and the reference device 200. At block 1518 of Fig. 13, the new wearable device 100 is paced by the further user at a known location on a body and orientated. While the wearable device 100 is orientated, the orientation of the wearable device 100 is determined (or measured) in the manner described earlier with reference to Figs. 5 or 11. The orientation of the wearable device 100 may be determined (or measured) continuously.

At block 1520 of Fig. 13, a feedback component is controlled to render (or output or provide) an output indicative of whether the wearable device 100 is in the predetermined orientation on the body 302, 402, 702, 802. For example, as described earlier, the output may provide an indication that informs a user to stop orientating the wearable device 100 when the predetermined orientation is reached (or achieved). At block 1522 of Fig. 13, the new wearable device 100 is placed on (and, for example, attached to) the body by the further user at the predetermined orientation.

Therefore, there is provided herein an improved method and device for determining an orientation of a wearable device.

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. The invention is defined by appended claims 1-15. Any reference signs in the claims should not be construed as limiting the scope.

## Claims

1. A wearable device (100) operable to determine an orientation of the wearable device (100), when placed on a body (302, 402), the wearable device (100) comprising:

   - a plurality of electrode pairs ($102_1$, $102_2$, $102_3$, $102_4$) located at different positions, wherein at least two of the plurality of electrode pairs ($102_1$, $102_2$, $102_3$, $102_4$) are configured to receive a signal transmitted from a reference device (200) using the body (302, 402) as a transmission medium; and
   - a processor (104) configured to:

     - determine a property associated with the signal received at the at least two electrode pairs;
     - compare the determined property to a corresponding reference property, wherein the determined property is indicative of an orientation of the wearable device (100) with respect to the reference device (200) and the corresponding reference property is indicative of a predetermined orientation for the wearable device (100) on the body (302, 402); and
     - determine, from the comparison, whether the wearable device (100) is in the predetermined orientation on the body (302, 402).

2. The wearable device (100) as claimed in claim 1, wherein the determined property associated with the signal received at the at least two electrode pairs ($102_1$, $102_2$, $102_3$, $102_4$) is indicative of a distance of one of the at least two electrode pairs ($102_1$, $102_2$, $102_3$, $102_4$) from the reference device (200) relative to a distance of at least one other of the at least two electrode pairs ($102_1$, $102_2$, $102_3$, $102_4$) from the reference device (200).

3. The wearable device (100) as claimed in claim 1 or 2, wherein the property associated with the signal received at the at least two electrode pairs ($102_1$, $102_2$, $102_3$, $102_4$) comprises any one or more of:

   - a phase angle difference between the signal received at the at least two electrode pairs ($102_1$, $102_2$, $102_3$, $102_4$);
   - a time of flight of the signal received at one of the at least two electrode pairs ($102_1$, $102_2$, $102_3$, $102_4$) relative to a time of flight of the signal received at at least one other of the at least two electrode pairs; and
   - an amplitude of the signal received at one of the at least two electrode pairs ($102_1$, $102_2$, $102_3$, $102_4$) relative to an amplitude of the signal received at at least one other of the at least two electrode pairs ($102_1$, $102_2$, $102_3$, $102_4$).

4. The wearable device (100) as claimed in any one of the preceding claims, wherein the wearable device (100) is arranged to be time synchronised with the reference device (200) prior to the transmission of the signal from the reference device (200).

**5.** The wearable device (100) as claimed in any one of the preceding claims, wherein the processor (104) is further configured to:
control a feedback component to render an output indicative of whether the wearable device (100) is in the predetermined orientation on the body.

**6.** The wearable device (100) as claimed in any one of the preceding claims, wherein the wearable device (100) further comprises:
at least one physiological characteristic sensor configured to obtain at least one physiological characteristic signal from the body (302, 402).

**7.** A method (500) of operating a wearable device (100) to determine an orientation of the wearable device (100), when placed on a body (302, 402), the wearable device (100) comprising a plurality of electrode pairs ($102_1$, $102_2$, $102_3$, $102_4$) located at different positions and the method (500) comprising:

- receiving (502), at at least two of the plurality of electrode pairs ($102_1$, $102_2$, $102_3$, $102_4$), a signal transmitted from a reference device using the body (302, 402) as a transmission medium;
- determining (504), by a processor (104) of the wearable device (100), a property associated with the signal received at the at least two electrode pairs ($102_1$, $102_2$, $102_3$, $102_4$);
- comparing (506), by the processor, the determined property to a corresponding reference property, wherein the determined property is indicative of an orientation of the wearable device (100) with respect to the reference device (200) and the corresponding reference property is indicative of a predetermined orientation for the wearable device (100) on the body (302, 402); and
- determining (508), by the processor (104), from the comparison, whether the wearable device (100) is in the predetermined orientation on the body (302, 402).

**8.** A reference device (200) operable to determine an orientation of a wearable device (100), when the wearable device (100) is placed on a body (702, 802), the wearable device (100) comprising a plurality of electrode pairs ($102_1$, $102_2$, $102_3$, $102_4$) located at different positions and the reference device (200) comprising:

- a receiver (202) configured to receive signals transmitted from at least two of the plurality of

electrode pairs ($102_1$, $102_2$, $102_3$, $102_4$) of the wearable device (100) through the body (702, 802) as a transmission medium; and
- a processor (204) configured to:

  - determine a property associated with the signals received at the receiver (202) from the at least two electrode pairs ($102_1$, $102_2$, $102_3$, $102_4$);
  - compare the determined property to a corresponding reference property, wherein the determined property is indicative of an orientation of the wearable device (100) with respect to the reference device (200) and the corresponding reference property is indicative of a predetermined orientation for the wearable device (100) on the body (702, 802); and
  - determine, from the comparison, whether the wearable device (100) is in the predetermined orientation on the body (702, 802).

**9.** The reference device (200) as claimed in claim 8, wherein the determined property associated with the signal received at the receiver (202) is indicative of a distance of one of the at least two electrode pairs ($102_1$, $102_2$, $102_3$, $102_4$) from the reference device (200) relative to a distance of at least one other of the at least two electrode pairs from the reference device.

**10.** The reference device (200) as claimed in claim 8 or 9, wherein the property associated with the signals received at the receiver (202) from the at least two electrode pairs ($102_1$, $102_2$, $102_3$, $102_4$) comprises any one or more of:

- a phase angle difference between the signals received at the receiver (202) from the at least two electrode pairs ($102_1$, $102_2$, $102_3$, $102_4$);
- a time of flight of the signal received at the receiver (202) from one of the at least two electrode pairs ($102_1$, $102_2$, $102_3$, $102_4$) relative to a time of flight of the signal received at the receiver (200) from at least one other of the at least two electrode pairs; and
- an amplitude of the signal received at the receiver (202) from one of the at least two electrode pairs ($102_1$, $102_2$, $102_3$, $102_4$) relative to an amplitude of the signal received at the receiver (200) from at least one other of the at least two electrode pairs.

**11.** The reference device (200) as claimed in any one of claims 8, 9 or 10, wherein the reference device (200) is time synchronised with the wearable device (100) prior to the transmission of the signals from the at least two electrode pairs ($102_1$, $102_2$, $102_3$, $102_4$).

**12.** The reference device (200) as claimed in any one of claims 8, 9, 10 or 11, wherein the processor (204) is further configured to:
control a feedback component to render an output indicative of whether the wearable device (100) is in a predefined orientation on the body (702, 802).

**13.** The reference device (200) as claimed in any one of the claims 8, 9, 10, 11 or 12, wherein the reference device (200) is a device contactable with at least part of the body (702, 802).

**14.** A method (900) of operating a reference device (200) to determine an orientation of a wearable device (100), when the wearable device (100) is placed on a body (702, 802), the wearable device (100) comprising a plurality of electrode pairs ($102_1$, $102_2$, $102_3$, $102_4$) located at different positions and the method (900) comprising:

- receiving (902), at a receiver (202) of the reference device (200), signals transmitted from at least two of the plurality of electrode pairs ($102_1$, $102_2$, $102_3$, $102_4$) of the wearable device (100) through the body (702, 802) as a transmission medium;
- determining (904), by a processor (204) of the reference device (200), a property associated with the signals received at the receiver (202) from the at least two electrode pairs ($102_1$, $102_2$, $102_3$, $102_4$);
- comparing (906), by the processor (204), the determined property to a corresponding reference property, wherein the determined property is indicative of an orientation of the wearable device (100) with respect to the reference device (200) and the corresponding reference property is indicative of a predetermined orientation for the wearable device (100) on the body (702, 802); and
- determining (910), by the processor (204), from the comparison, whether the wearable device (100) is in the predetermined orientation on the body (702, 802).

**15.** A system (300, 400, 700, 800) comprising:

- the wearable device (100) as claimed in any one of claims 1 to 6 and the reference device configured to transmit the signal; or
- the reference device (200) as claimed in any one of claims 8 to 13 and the wearable device comprising the at least two electrodes configured to transmit the signals.

**Patentansprüche**

**1.** Ein tragbares Gerät (100) zum Ermitteln der Ausrichtung des an einem Körper (302, 402) angebrachten tragbaren Geräts (100), wobei das tragbare Gerät (100) Folgendes umfasst:

- mehrere Elektrodenpaare ($102_1$, $102_2$, $102_3$, $102_4$), die sich an unterschiedlichen Positionen befinden, wobei mindestens zwei der Elektrodenpaare ($102_1$, $102_2$, $102_3$, $102_4$) ein unter Verwendung des Körpers (302, 402) als Übertragungsmedium von einem Referenzgerät (200) gesendetes Signal empfangen; und
- einen Prozessor (104), der folgende Schritte durchführt:

- Ermitteln einer Eigenschaft, die dem von den mindestens zwei Elektrodenpaaren empfangenen Signal zugeordnet ist;
- Vergleichen der ermittelten Eigenschaft mit einer entsprechenden Referenzeigenschaft, wobei die ermittelte Eigenschaft die Ausrichtung des tragbaren Geräts (100) in Bezug auf das Referenzgerät (200) und die entsprechende Referenzeigenschaft eine vorab festgelegte Ausrichtung des tragbaren Geräts (100) am Körper (302, 402) angibt; und
- anhand des Vergleichs Ermitteln, ob sich das tragbare Gerät (100) in der vorab festgelegten Ausrichtung am Körper (302, 402) befindet.

**2.** Das tragbare Gerät (100) gemäß Anspruch 1, wobei die ermittelte, dem von den mindestens zwei Elektrodenpaaren ($102_1$, $102_2$, $102_3$, $102_4$) empfangenen Signal zugeordnete Eigenschaft den Abstand eines der mindestens zwei Elektrodenpaare ($102_1$, $102_2$, $102_3$, $102_4$) zum Referenzgerät (200) relativ zum Abstand mindestens eines anderen der mindestens zwei Elektrodenpaare ($102_1$, $102_2$, $102_3$, $102_4$) zum Referenzgerät (200) angibt.

**3.** Das tragbare Gerät (100) gemäß Anspruch 1 oder 2, wobei die dem von den mindestens zwei Elektrodenpaaren ($102_1$, $102_2$, $102_3$, $102_4$) empfangenen Signal zugeordnete Eigenschaft mindestens eine der folgenden Eigenschaften umfasst:

- eine Phasenwinkeldifferenz zwischen dem von den mindestens zwei Elektrodenpaaren ($102_1$, $102_2$, $102_3$, $102_4$) empfangenen Signal;
- die Laufzeit des von einem der mindestens zwei Elektrodenpaare ($102_1$, $102_2$, $102_3$, $102_4$) empfangenen Signals relativ zur Laufzeit des von mindestens einem anderen der mindestens zwei Elektrodenpaare empfangenen Signals;

und

- die Amplitude des von einem der mindestens zwei Elektrodenpaare ($102_1$, $102_2$, $102_3$, $102_4$) empfangenen Signals relativ zur Amplitude des von mindestens einem anderen der mindestens zwei Elektrodenpaare ($102_1$, $102_2$, $102_3$, $102_4$) empfangenen Signals.

4. Ein tragbares Gerät (100) gemäß einem der vorherigen Ansprüche, wobei das tragbare Gerät (100) vor dem Übertragen des Signals vom Referenzgerät (200) mit dem Referenzgerät (200) zeitlich synchronisiert wird.

5. Ein tragbares Gerät (100) gemäß einem der vorherigen Ansprüche, wobei der Prozessor (104) zudem folgenden Schritt durchführt:
Steuern einer Feedback-Komponente, um eine Ausgabe bereitzustellen, die angibt, ob sich das tragbare Gerät (100) in der vorab festgelegten Ausrichtung am Körper befindet.

6. Ein tragbares Gerät (100) gemäß einem der vorherigen Ansprüche, wobei das tragbare Gerät (100) zudem Folgendes umfasst:
mindestens einen Sensor für physiologische Eigenschaften, der mindestens ein physiologisches Eigenschaftssignal vom Körper (302, 402) abruft.

7. Eine Methode (500) zum Betreiben eines tragbaren Geräts (100) zum Ermitteln der Ausrichtung des an einem Körper (302, 402) angebrachten tragbaren Geräts (100), wobei das tragbare Gerät (100) mehrere Elektrodenpaare ($102_1$, $102_2$, $102_3$, $102_4$) umfasst, die sich an unterschiedlichen Positionen befinden, und wobei die Methode (500) folgende Schritte umfasst:

- Abrufen (502) mit mindestens zwei der Elektrodenpaare ($102_1$, $102_2$, $102_3$, $102_4$) eines unter Verwendung des Körpers (302, 402) als Übertragungsmedium von einem Referenzgerät gesendeten Signals;
- mit einem Prozessor (104) des tragbaren Geräts (100) Ermitteln (504) einer Eigenschaft, die dem von den mindestens zwei Elektrodenpaaren ($102_1$, $102_2$, $102_3$, $102_4$) empfangenen Signal zugeordnet ist;
- mit dem Prozessor Vergleichen (506) der ermittelten Eigenschaft mit einer entsprechenden Referenzeigenschaft, wobei die ermittelte Eigenschaft die Ausrichtung des tragbaren Geräts (100) in Bezug auf das Referenzgerät (200) und die entsprechende Referenzeigenschaft eine vorab festgelegte Ausrichtung des tragbaren Geräts (100) am Körper (302, 402) angibt; und
- anhand (508) des Vergleichs mit dem Prozessor (104) Ermitteln, ob sich das tragbare Gerät (100) in der vorab festgelegten Ausrichtung am Körper (302, 402) befindet.

8. Ein Referenzgerät (200) zum Ermitteln der Ausrichtung eines tragbaren Geräts (100), wenn das tragbare Gerät (100) an einem Körper (702, 802) angebracht ist, wobei das tragbare Gerät (100) mehrere Elektrodenpaare ($102_1$, $102_2$, $102_3$, $102_4$) umfasst, die sich an unterschiedlichen Positionen befinden, und wobei das Referenzgerät (200) Folgendes umfasst:

- einen Empfänger (202) zum Empfangen von Signalen, die von mindestens zwei der Elektrodenpaare ($102_1$, $102_2$, $102_3$, $102_4$) des tragbaren Geräts (100) mithilfe des Körpers (702, 802) als Übertragungsmedium übertragen werden; und
- einen Prozessor (204), der für folgende Schritte konfiguriert ist:

- Ermitteln einer Eigenschaft, die den vom Empfänger (202) von den mindestens zwei Elektrodenpaaren ($102_1$, $102_2$, $102_3$, $102_4$) abgerufenen Signalen zugeordnet ist;
- Vergleichen der ermittelten Eigenschaft mit einer entsprechenden Referenzeigenschaft, wobei die ermittelte Eigenschaft die Ausrichtung des tragbaren Geräts (100) in Bezug auf das Referenzgerät (200) und die entsprechende Referenzeigenschaft eine vorab festgelegte Ausrichtung des tragbaren Geräts (100) am Körper (702, 802) angibt; und
- anhand des Vergleichs Ermitteln, ob sich das tragbare Gerät (100) in der vorab festgelegten Ausrichtung am Körper (702, 802) befindet.

9. Das Referenzgerät (200) gemäß Anspruch 8, wobei die ermittelte, dem vom Empfänger (202) empfangenen Signal zugeordnete Eigenschaft den Abstand eines der mindestens zwei Elektrodenpaare ($102_1$, $102_2$, $102_3$, $102_4$) zum Referenzgerät (200) relativ zum Abstand mindestens eines anderen der mindestens zwei Elektrodenpaare zum Referenzgerät angibt.

10. Das Referenzgerät (200) gemäß Anspruch 8 oder 9, wobei die den vom Empfänger (202) von den mindestens zwei Elektrodenpaaren ($102_1$, $102_2$, $102_3$, $102_4$) abgerufenen Signale zugeordnete Eigenschaft mindestens eine der folgenden Eigenschaften umfasst:

- eine Phasenwinkeldifferenz zwischen den vom Empfänger (202) von den mindestens zwei Elektrodenpaaren ($102_1$, $102_2$, $102_3$, $102_4$) ab-

gerufenen Signalen;
- die Laufzeit des vom Empfänger (202) von einem der mindestens zwei Elektrodenpaare ($102_1$, $102_2$, $102_3$, $102_4$) abgerufenen Signals relativ zur Laufzeit des vom Empfänger (200) von mindestens einem anderen der mindestens zwei Elektrodenpaare empfangenen Signals; und
- die Amplitude des vom Empfänger (202) von einem der mindestens zwei Elektrodenpaare ($102_1$, $102_2$, $102_3$, $102_4$) abgerufenen Signals relativ zur Amplitude des vom Empfänger (200) von mindestens einem anderen der mindestens zwei Elektrodenpaare empfangenen Signals.

11. Das Referenzgerät (200) gemäß einem der Ansprüche 8, 9 oder 10, wobei das Referenzgerät (200) vor dem Übertragen der Signale von den mindestens zwei Elektrodenpaaren ($102_1$, $102_2$, $102_3$, $102_4$) mit dem tragbaren Gerät (100) zeitlich synchronisiert wird.

12. Das Referenzgerät (200) gemäß einem der Ansprüche 8, 9, 10 oder 11, wobei der Prozessor (204) zudem folgenden Schritt durchführt:
Steuern einer Feedback-Komponente, um eine Ausgabe bereitzustellen, die angibt, ob sich das tragbare Gerät (100) in der vorab festgelegten Ausrichtung am Körper (702, 802) befindet.

13. Das Referenzgerät (200) gemäß einem der Ansprüche 8, 9, 10, 11 oder 12, wobei das Referenzgerät (200) mit zumindest einem Teil des Körpers (702, 802) in Kontakt gebracht werden kann.

14. Eine Methode (900) zum Betreiben eines Referenzgeräts (200) zum Ermitteln der Ausrichtung eines tragbaren Geräts (100), wenn das tragbare Gerät (100) an einem Körper (702, 802) angebracht ist, wobei das tragbare Gerät (100) mehrere Elektrodenpaare ($102_1$, $102_2$, $102_3$, $102_4$) umfasst, die sich an unterschiedlichen Positionen befinden, und wobei die Methode (900) folgende Schritte umfasst:

- mit einem Empfänger (202) des Referenzgeräts (200) Empfangen (902) von Signalen, die von mindestens zwei der Elektrodenpaare ($102_1$, $102_2$, $102_3$, $102_4$) des tragbaren Geräts (100) mithilfe des Körpers (702, 802) als Übertragungsmedium übertragen werden;
- mit einem Prozessor (204) des Referenzgeräts (200) Ermitteln (904) einer Eigenschaft, die den vom Empfänger (202) von den mindestens zwei Elektrodenpaaren ($102_1$, $102_2$, $102_3$, $102_4$) empfangenen Signalen zugeordnet ist;
- mit dem Prozessor (204) Vergleichen (906) der ermittelten Eigenschaft mit einer entsprechenden Referenzeigenschaft, wobei die ermittelte Eigenschaft die Ausrichtung des tragbaren Geräts (100) in Bezug auf das Referenzgerät (200) und die entsprechende Referenzeigenschaft eine vorab festgelegte Ausrichtung des tragbaren Geräts (100) am Körper (702, 802) angibt; und
- anhand des Vergleichs mit dem Prozessor (204) Ermitteln (910), ob sich das tragbare Gerät (100) in der vorab festgelegten Ausrichtung am Körper (702, 802) befindet.

15. Ein System (300, 400, 700, 800), das Folgendes umfasst:

- das tragbare Gerät (100) gemäß einem der Ansprüche 1 bis 6 sowie das Referenzgerät zum Senden des Signals; oder
- das Referenzgerät (200) gemäß einem der Ansprüche 8 bis 13 sowie das tragbare Gerät mit den mindestens zwei Elektroden zum Übertragen der Signale.

**Revendications**

1. Dispositif (100) portable utilisable pour déterminer une orientation du dispositif (100) portable lorsqu'il est placé sur un corps (302, 402), le dispositif (100) portable comprenant :

- une pluralité de paires d'électrodes ($102_1$, $102_2$, $102_3$, $102_4$) situées à des positions différentes, dans lequel les au moins deux paires de la pluralité de paires d'électrodes ($102_1$, $102_2$, $102_3$, $102_4$) sont conçues pour recevoir un signal transmis depuis un dispositif de référence (200) utilisant le corps (302, 402) comme support de transmission ; et
- un processeur (104) configuré :

- pour déterminer une propriété associée au signal reçu au niveau des au moins deux paires d'électrodes ;
- pour comparer la propriété déterminée à une propriété de référence correspondante, dans lequel la propriété déterminée indique une orientation du dispositif (100) portable par rapport au dispositif de référence (200) et la propriété de référence correspondante indique une orientation prédéterminée pour le dispositif (100) portable sur le corps (302, 402) ; et
- pour déterminer, à partir de la comparaison, si le dispositif (100) portable est positionné selon l'orientation prédéterminée sur le corps (302, 402).

2. Dispositif (100) portable selon la revendication 1, dans lequel la propriété déterminée associée au signal reçu au niveau des au moins deux paires d'électrodes ($102_1$, $102_2$, $102_3$, $102_4$) indique une distance de l'une des au moins deux paires d'électrodes ($102_1$, $102_2$, $102_3$, $102_4$) du dispositif de référence (200) par rapport à une distance de l'au moins une autre des au moins deux paires d'électrodes ($102_1$, $102_2$, $102_3$, $102_4$) du dispositif de référence (200).

3. Dispositif (100) portable selon la revendication 1 ou 2, dans lequel la propriété associée au signal reçu au niveau des au moins deux paires d'électrodes ($102_1$, $102_2$, $102_3$, $102_4$) comprend :

    - une différence d'angle de phase entre le signal reçu au niveau des au moins deux paires d'électrodes ($102_1$, $102_2$, $102_3$, $102_4$) ; et/ou
    - un temps de vol du signal reçu sur l'une des au moins deux paires d'électrodes ($102_1$, $102_2$, $102_3$, $102_4$) par rapport à un temps de vol du signal reçu sur l'au moins une autre des au moins deux paires d'électrodes ; et/ou
    - une amplitude du signal reçu sur l'une des au moins deux paires d'électrodes ($102_1$, $102_2$, $102_3$, $102_4$) par rapport à une amplitude du signal reçu sur l'au moins une autre des au moins deux paires d'électrodes ($102_1$, $102_2$, $102_3$, $102_4$).

4. Dispositif (100) portable selon l'une quelconque des revendications précédentes, dans lequel le dispositif (100) portable est conçu pour être synchronisé dans le temps avec le dispositif de référence (200) avant la transmission du signal depuis le dispositif de référence (200).

5. Dispositif (100) portable selon l'une quelconque des revendications précédentes, dans lequel le processeur (104) est en outre configuré :
pour commander un composant de rétroaction pour rendre une sortie indiquant si le dispositif (100) portable est positionné selon l'orientation prédéterminée sur le corps.

6. Dispositif (100) portable selon l'une quelconque des revendications précédentes, dans lequel le dispositif (100) portable comprend en outre :
au moins un capteur de caractéristique physiologique conçu pour obtenir au moins un signal de caractéristique physiologique du corps (302, 402).

7. Procédé (500) de fonctionnement d'un dispositif (100) portable pour déterminer une orientation du dispositif (100) portable, lorsqu'il est placé sur un corps (302, 402), le dispositif (100) portable comprenant une pluralité de paires d'électrodes $102_2$, $102_3$, situés à différentes positions et le procédé (500) comprenant :

    - la réception (502), au niveau d'au moins deux de la pluralité de paires d'électrodes ($102_1$, $102_2$, $102_3$, $102_4$), d'un signal transmis depuis un dispositif de référence utilisant le corps (302, 402) comme support de transmission ;
    - la détermination (504), par un processeur (104) du dispositif (100) portable, d'une propriété associée au signal reçu au niveau des au moins deux paires d'électrodes ($102_1$, $102_2$, $102_3$, ;
    - la comparaison (506), par le processeur, de la propriété déterminée à une propriété de référence correspondante, dans lequel la propriété déterminée indique une orientation du dispositif (100) portable par rapport au dispositif de référence (200) et la propriété de référence correspondante indique une orientation prédéterminée pour le dispositif (100) portable sur le corps (302, 402) ; et
    - la détermination (508), par le processeur (104), à partir de la comparaison, si le dispositif (100) portable est positionné selon l'orientation prédéterminée sur le corps (302, 402).

8. Dispositif de référence (200) utilisable pour déterminer une orientation d'un dispositif (100) portable lorsque le dispositif (100) portable est placé sur un corps (702, 802), le dispositif (100) portable comprenant une pluralité de paires d'électrodes ($102_1$, $102_2$, $102_3$, situés à différentes positions et le dispositif de référence (200) comprenant :

    - un récepteur (202) conçu pour recevoir des signaux transmis depuis les au moins deux de la pluralité de paires d'électrodes ($102_1$, $102_2$, $102_3$, du dispositif (100) portable à travers le corps (702, 802) comme support de transmission ; et

un processeur (204) configuré :

    - pour déterminer une propriété associée aux signaux reçus au niveau du récepteur (202) des au moins deux paires d'électrodes ($102_1$, $102_2$, $102_3$, ;
    - pour comparer la propriété déterminée à une propriété de référence correspondante, la propriété déterminée indiquant une orientation du dispositif (100) portable par rapport au dispositif de référence (200) et la propriété de référence correspondante indiquant une orientation prédéterminée pour le dispositif (100) portable sur le corps (702, 802) ; et
    - pour déterminer, à partir de la comparaison, si le dispositif (100) portable est positionné selon l'orientation prédéterminée sur le corps (702, 802).

9. Dispositif de référence (200) selon la revendication

**EP 3 684 245 B1**

8, dans lequel la propriété déterminée associée au signal reçu au niveau du récepteur (202) indique une distance de l'une des au moins deux paires d'électrodes (102₁, 102₂, 102₃, du dispositif de référence (200) par rapport à une distance de l'au moins une autre des au moins deux paires d'électrodes du dispositif de référence.

10. Dispositif de référence (200) selon la revendication 8 ou 9, dans lequel la propriété associée aux signaux reçus au niveau du récepteur (202) des au moins deux paires d'électrodes (102₁, 102₂, 102₃, comprend :

- une différence d'angle de phase entre les signaux reçus au niveau du récepteur (202) des au moins deux paires d'électrodes (102₁, 102₂, 102₃, 102₄) ; et/ou
- un temps de vol du signal reçu au niveau du récepteur (202) de l'une des au moins deux paires d'électrodes (102₁, 102₂, 102₃, 102₄) par rapport à un temps de vol du signal reçu au niveau du récepteur (200) de l'au moins une autre des au moins deux paires d'électrodes ; et/ou
- une amplitude du signal reçu au niveau du récepteur (202) de l'une des au moins deux paires d'électrodes (102₁, 102₂, 102₃, 102₄) par rapport à une amplitude du signal reçu au niveau du récepteur (200) de l'au moins une autre des au moins deux paires d'électrodes.

11. Dispositif de référence (200) selon l'une quelconque des revendications 8, 9 ou 10, dans lequel le dispositif de référence (200) est synchronisé dans le temps avec le dispositif (100) portable avant la transmission des signaux provenant des au moins deux paires d'électrodes (102₁, 102₂, 102₃, 102₄).

12. Dispositif de référence (200) selon l'une quelconque des revendications 8, 9, 10 ou 11, dans lequel le processeur (204) est en outre configuré :
pour commander un composant de rétroaction pour rendre une sortie indiquant si le dispositif (100) portable est positionné selon l'orientation prédéterminée sur le corps (702, 802).

13. Dispositif de référence (200) selon l'une quelconque des revendications 8, 9, 10, 11 ou 12, dans lequel le dispositif de référence (200) est un dispositif pouvant entrer en contact avec au moins une partie du corps (702, 802).

14. Procédé (900) de fonctionnement d'un dispositif de référence (200) pour déterminer une orientation d'un dispositif (100) portable lorsque le dispositif (100) portable est placé sur un corps (702, 802), le dispositif (100) portable comprenant une pluralité de paires d'électrodes (102₁, 102₂, 102₃, 102₄) situées à différentes positions et le procédé (900) comprenant :

- la réception (902), au niveau d'un récepteur (202) du dispositif de référence (200), des signaux transmis depuis les au moins deux de la pluralité de paires d'électrodes (102₁, 102₂, 102₃, 102₄) du dispositif (100) portable par l'intermédiaire du corps (702, 802) comme support de transmission ;
- la détermination (904), par un processeur (204) du dispositif de référence (200), d'une propriété associée aux signaux reçus au niveau du récepteur (202) des au moins deux paires d'électrodes (102₁, 102₂, 102₃, 102₄) ;
- la comparaison (906), par le processeur (204), de la propriété déterminée à une propriété de référence correspondante, dans lequel la propriété déterminée indique une orientation du dispositif (100) portable par rapport au dispositif de référence (200) et la propriété de référence correspondante indique une orientation prédéterminée pour le dispositif (100) portable sur le corps (702, 802) ; et
- la détermination (910), par le processeur (204), à partir de la comparaison, si le dispositif (100) portable est positionné selon l'orientation prédéterminée sur le corps (702, 802).

15. Système (300, 400, 700, 800) comprenant :

- le dispositif (100) portable selon l'une quelconque des revendications 1 à 6 et le dispositif de référence conçu pour transmettre le signal ; ou
- le dispositif de référence (200) selon l'une quelconque des revendications 8 à 13 et le dispositif portable comprenant les au moins deux électrodes conçues pour transmettre les signaux.

Figure 1

Figure 2

EP 3 684 245 B1

Figure 3

402

100

100

400

200

200

Figure 4

500

Receive, at at least two electrode pairs
of the wearable device, signal
transmitted from reference device
using body as transmission medium

502

Determine property associated with signal
received at the at least two electrode pairs

504

Compare determined property to
corresponding reference property

506

From the comparison, determine
whether wearable device is in
predetermined orientation on body

508

Figure 5

Figure 6

Syncronised Internal Clocks

Transmitted signal

Reference signal

Received signal

$\Delta t$

Time

Figure 7

Figure 8

Figure 9

Figure 10

900

```
┌─────────────────────────────────────┐
│  Receive, at a receiver of the reference │
│  device, signals transmitted from at least │
902 │  two electrode pairs of wearable device │
│  through body as transmission medium │
└─────────────────────────────────────┘
                    │
                    ▼
┌─────────────────────────────────────┐
│  Determine property associated with │
│  signals received at receiver from │
904 │  the at least two electrode pairs │
└─────────────────────────────────────┘
                    │
                    ▼
┌─────────────────────────────────────┐
│  Compare determined property to │
906 │  corresponding reference property │
└─────────────────────────────────────┘
                    │
                    ▼
┌─────────────────────────────────────┐
│  From the comparison, determine │
│  whether wearable device is in │
908 │  predetermined orientation on body │
└─────────────────────────────────────┘
```

Figure 11

```
┌─────────────────────────────────────┐
│      Run application to configure    │
1002 │          wearable device             │
│                                      │
└─────────────────────────────────────┘
                   │
                   ▼
┌─────────────────────────────────────┐
│   Place wearable device in at least one │
1004 │     predetermined orientation on body │
└─────────────────────────────────────┘
                   │
                   ▼
┌─────────────────────────────────────┐
│      For the at least one predetermined │
1006 │  orientation, acquire orientation coordinates │
└─────────────────────────────────────┘
                   │
                   ▼
┌─────────────────────────────────────┐
│            End configuration          │
1008 │                                      │
└─────────────────────────────────────┘
```

1000

```
                   │
                   ▼
┌─────────────────────────────────────┐
│       Run application to guide placement │
1012 │          of wearable device           │
└─────────────────────────────────────┘
                   │
                   ▼
┌─────────────────────────────────────┐
│   Place new wearable device at a known │
│   location and orientate while orientation │
1014 │              measured                  │
└─────────────────────────────────────┘
                   │
                   ▼
┌─────────────────────────────────────┐
│       Feedback whether predetermined  │
1016 │         orientation achieved          │
└─────────────────────────────────────┘
                   │
                   ▼
┌─────────────────────────────────────┐
│     Attach the new wearable device at the │
1018 │       predetermined orientation        │
└─────────────────────────────────────┘
```

1010

Figure 12

```
┌─────────────────────────────────────┐
│        Run application to configure  │
│            wearable device           │
│  1502                                │
└─────────────────────────────────────┘
                  │
                  ▼
┌─────────────────────────────────────┐
│   Synchronise internal clocks of wearable │
│       device and reference device    │
│  1504                                │
└─────────────────────────────────────┘
                  │
                  ▼
┌─────────────────────────────────────┐
│    Place wearable device in at least one │
│      predetermined orientation on body │
│  1506                                │
└─────────────────────────────────────┘
                  │
                  ▼
┌─────────────────────────────────────┐
│  For the at least one predetermined orientation, │
│         acquire orientation coordinates │
│  1508                                │
└─────────────────────────────────────┘
                  │
                  ▼
┌─────────────────────────────────────┐
│           End configuration          │
│  1510                                │
└─────────────────────────────────────┘
                  │
                  ▼
┌─────────────────────────────────────┐
│      Run application to guide placement │
│           of wearable device         │
│  1514                                │
└─────────────────────────────────────┘
                  │
                  ▼
┌─────────────────────────────────────┐
│   Synchronise internal clocks of wearable │
│       device and reference device    │
│  1516                                │
└─────────────────────────────────────┘
                  │
                  ▼
┌─────────────────────────────────────┐
│    Place the new wearable device at a known │
│  location and orientate while orientation measured │
│  1518                                │
└─────────────────────────────────────┘
                  │
                  ▼
┌─────────────────────────────────────┐
│      Feedback whether predetermined  │
│          orientation achieved        │
│  1520                                │
└─────────────────────────────────────┘
                  │
                  ▼
┌─────────────────────────────────────┐
│     Attach the new wearable device at the │
│        predetermined orientation     │
│  1522                                │
└─────────────────────────────────────┘
```

1500

1512

Figure 13

**EP 3 684 245 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016181321 A1
- US 20130338448 A1
- US 20130116533 A1